# EUROPEAN PATENT SPECIFICATION

(11) **EP 3 224 606 B1**
(45) Date of publication and mention of the grant of the patent: **28.08.2019**
(21) Application number: 15804218.4
(22) Date of filing: 27.11.2015
(51) Int. Cl.: G01N 27/02, G01N 15/06, G01N 33/28, G01N 15/10, G01N 27/74, G01N 15/02, G01N 15/00

(54) **SENSOR APPARATUS**
SENSORVORRICHTUNG
DISPOSITIF DÉTECTEUR

(30) Priority: 28.11.2014 GB 201421219; 19.10.2015 GB 201518471
(43) Date of publication of application: 04.10.2017
(60) Divisional application: 18155034.4
(73) Proprietor: Parker Hannifin Manufacturing Limited, Hemel Hempstead, Hertfordshire HP2 4SJ (GB)
(72) Inventor: FOORD, Anthony Peter, Worthing, Sussex BN14 9NP (GB)
(74) Representative: Dehns
(86) International application number: PCT/GB2015/053639
(87) International publication number: WO 2016/083839

(56) References cited:
- CN-A- 102 243 200
- CN-A- 103 674 787
- US-A- 3 748 576
- US-A1- 2009 051 350
- US-A1- 2009 112 507
- US-A1- 2010 126 251
- US-A1- 2014 144 216
- DU L ET AL: "A high throughput inductive pulse sensor for online oil debris monitoring", TRIBOLOGY INTERNATIONAL, BUTTERWORTH SCIENTIFIC LDT, GUILDFORD, GB, vol. 44, no. 2, 29 October 2010 (2010-10-29), pages 175-179, XP027525878, ISSN: 0301-679X [retrieved on 2010-10-29]

## Description

The present invention is defined by the appended claims and relates to a sensor apparatus for use in obtaining information relating to the presence of metal particles within a sample of a substance to be tested. The present invention also relates to methods of using the sensor apparatus and system.

The ability to obtain information relating to the presence of metal particles in a sample of a substance e.g. a sample of fluid finds application in a variety of fields. The information may be indicative simply of whether metal particles are present, or may comprise more detailed information e.g. as to a quantity of the particles, and/or properties of the particles. The apparatus and methods described herein are particularly, although not exclusively, suitable for detecting or monitoring machine wear debris, e.g. detecting or monitoring the content with respect to volume e.g. per unit volume, size and/or type of metal particles present in lubricating oil or grease from an engine, turbine or gear box. In normal use, the moving parts of an engine, turbine or gear box etc. will generate relatively small (e.g. less than 10 microns) metal particles. However, an increase in the rate at which such debris is generated, or a change in the size or type of metal particles being generated, may indicate an engine, turbine or gear box etc. fault, or damage to a part thereof. The type of particles being generated may even be diagnostic of a faulty component. Of course, the present invention may be applied to any other applications in which it is desired to obtain information relating to the presence of metal particles within a sample e.g. of fluid.

The present invention is concerned with the detection of metal particles using magnetic fields. All metals are electrically conductive, and some metals also exhibit some form of magnetic behaviour. For instance, metals having a spontaneous net magnetic moment, such as iron, nickel and cobalt, are classified as ferromagnetic. Ferromagnetic materials may in turn be classified as "soft" or "hard". Soft ferromagnets have a low coercivity, i.e. the net magnetisation can easily be reversed e.g. by changing the direction of an applied magnetic field. Hard ferromagnets on the other hand tend to retain more of their net magnetisation when an applied field is removed, or reversed, and their magnetisation can only be reversed by applying a relatively strong opposed magnetic field. Materials with no spontaneous magnetic moment may be classified e.g. as paramagnetic if the magnetic moments tend to align with an applied field, or diamagnetic if the magnetic moments tend to oppose an applied field. Other forms of magnetic order are also known, including ferrimagnetism, helical magnetism, and antiferromagnetism. In general, the magnetic behaviour of a material may be described through a combination of various factors.

Metal particles likely to be present in engine oil or grease will tend to be either mechanically hard ferrous materials, such as iron or steels used to form the moving parts or hard wearing surfaces (gears, etc.), or mechanically soft non-ferrous materials, such as lead, tin, brass, aluminium or white metal, which may be used to hold the parts together (housings, bearings, etc.). Ferrous materials are normally soft ferromagnets, and for the purposes of this application the term "ferrous" will be used to describe the class of ferromagnetic materials comprising i.e. containing or consisting of iron (consistent with how it would normally be understood). Although non-ferrous ferromagnets, e.g. nickel and cobalt, also exist, most non-ferrous materials are paramagnetic or diamagnetic. Many non-ferrous materials only show a negligible paramagnetic or diamagnetic response to a magnetic field, and may essentially be classed as "non-magnetic".

The general principles behind metal detection using a magnetic field are well established, and the skilled person will understand that, and how, different metal particles may interact with an applied magnetic field. The basic concept is that an alternating magnetic field induces eddy currents in any conducting material, and will also be affected by the magnetic properties of the material. By detecting a resulting change in the magnetic field it is therefore possible to detect the presence of metals. Analysis of the signal may also allow classification of the material e.g. as ferromagnetic or non-ferromagnetic and/or a quantity of the material to be determined. The skilled person will also be aware of various established coil arrangements that can be used for measuring magnetic and conductive properties of metal particles within a test substance e.g. fluid.

Known coil arrangements fall into two main groups: either there are separate coils for transmitting and receiving a magnetic field, or the same coil effectively acts as both transmitter and receiver. An example of the latter arrangement is described in US patent no. 6,051,970 (HUTCHINGS). In US 6,051,970 a solenoidal coil is used to generate a magnetic field and a change in impedance of this coil in response to a sample being introduced into the field is monitored to detect the presence of metal particles. The development described in US 6,051,970 is the coaxial mounting of the solenoidal coil within a ferrite sleeve flux guide. Further coil configurations are known from CN103674787, US2014/144216 and CN102243200.

As will be discussed further below the present inventors have recognised a number of problems associated with this sensor apparatus.

It is desired to provide an improved sensor apparatus, and in particular an improved coil arrangement for a sensor apparatus, for use in obtaining information relating to the presence of metal particles within a sample.

Accordingly, from a first aspect, the present invention provides a sensor apparatus for use in obtaining information relating to the presence of metal particles within a sample of a substance to be tested, as defined by independent claim 1.

It has been found that the present invention may provide a sensor apparatus for use in obtaining information relating to the presence of metal particles within a substance which can advantageously be manufactured with a good degree of reproducibility, and in a time- and cost-effective manner, at least in comparison to conventional such sensor apparatus which rely upon a wound solenoidal coil e.g. as described in US 6,051,970. This is because the apparatus includes a sensing coil which is a printed coil. According to the independent claims, the coil is a printed coil of a printed circuit board (PCB). Printing processes are generally cheap, and facilitate automation since the same pattern can easily be precisely reproduced. In contrast, conventional solenoidal coils must be formed by mechanical winding processes that require relatively expensive machinery and tend to be relatively time and/or labour intensive. Furthermore, such mechanical processes are more likely to suffer from a lack of reproducibility, and are more sensitive to changes in operating conditions than known printing processes. Conventional mechanical processes are thus more likely to result in mechanical imperfections that may reduce the sensitivity of the sensor, or at least result in variation in sensitivity obtained. Variation inherent in such processes may also result in a number of products being rejected during quality control checks (hence further increasing the cost or time of the manufacturing process). In general, the use of the printing process to provide the sensing coil in accordance with the present invention may enable a sensor to be provided with improved tolerances.

Also, because the sensing coil is printed on the substrate e.g. being fabricated as part of a printed circuit, the present coil arrangement may be relatively mechanically stable in use. That is, the substrate may provide mechanical support to the coil in use and the position of the coil is fixed precisely on the substrate, i.e. relative to the aperture. This may reduce the likelihood of the coil moving or microscopically changing in dimension in use which could lead to a variation e.g. reduction in sensitivity and/or resolution of the sensor. The sensor apparatus may therefore provide more reliable results.

It will be appreciated that the sensor apparatus of the present invention may provide advantages in terms of ease of manufacture, stability and reliability in comparison to a conventional mechanically wound coil as a result of the use of the printed sensor coil, whether or not it provides results of comparable sensitivity. In many applications such considerations may be more important than the sensitivity of the sensor. Thus, it may be acceptable to sacrifice some sensitivity of the sensor in view of other advantages provided. Nonetheless, it has been found that by choosing an appropriate substrate material and coil geometry the present sensor apparatus may provide at least a comparable sensitivity to conventional wound coils. In fact, at least for certain applications, the present coil sensor may provide a higher sensitivity and/or resolution, or allow a higher sample throughput, than conventional wound coil arrangements. The arrangement is particularly advantageous when the sensor apparatus is used to detect e.g. count and/or categorise individual metal particles within a dynamic sample e.g. a volume of fluid flowing through the aperture, e.g. within a test period.

These, and other, advantages that arise from embodiments of the present invention will be expanded upon in the discussion below. It will however be appreciated therefore that printing coils onto a substrate as described herein in providing a sensor apparatus for use in obtaining information relating to the presence of metal particles within a sample therefore provides a number of improvements over conventional mechanically wound coil arrangements like that described in US 6,051,970.

The sensing means senses the result of interaction between a magnetic field generated by the magnetic field generating means and the sample in use, i.e. when the magnetic field generating means is energised.

The sensing means may be seen as having a sensing region, the sensing means being arranged to sense the result of interaction between the generated magnetic field and the sample in the sensing region. As discussed in more detail below, the extent of the sensing region will depend upon the sensing means e.g. coil, and may also be influenced by the nature of the substance to be tested.

The sample of the substance to be tested in accordance with the present invention may be a static or a dynamic sample. In some embodiments the sample is a dynamic sample defined by a volume of fluid which flows through the aperture e.g. within a test period i.e. between a first time and a second time. In other embodiments the sample is a static sample i.e. an extracted volume of a substance to be tested.

The present invention also extends to a method of obtaining information relating to the presence of metal particles within a sample of a substance to be tested using the sensor apparatus of the present invention in accordance with any of the embodiments described herein. The method may comprise locating a sample of a substance to be tested such that it is received in said aperture; using the magnetic field generating means to generate a magnetic field; and using the sensing means to sense the result of interaction between the sample and the applied magnetic field. The sensed result of interaction may be a sensed result of interaction within a sensing region of the sensing means.

From yet another aspect, the present invention provides a method of obtaining information relating to the presence of metal particles within a sample using a sensor apparatus in accordance with the invention in any of its aspects or embodiments, the method comprising:
locating a sample of a substance to be tested such that the sample is received in said aperture;
using the magnetic field generating means to generate a magnetic field;
and using the sensing means to sense the result of interaction (e.g. within a sensing region of the sensing means) between the sample and the applied magnetic field.

In accordance with a further aspect of the present invention there is provided a method of obtaining information relating to the presence of metal particles within a sample, the method comprising providing a sensor apparatus comprising:
a substrate comprising an aperture for receiving a sample of a substance to be tested, the substrate having one or more electrically conductive coils printed thereon;
magnetic field generating means for generating a magnetic field for application to a sample received in the aperture; and
sensing means for sensing the result of interaction (e.g. within a sensing region of the sensing means) between the generated magnetic field and a sample received in the aperture, the sensed result of the interaction being usable to determine information relating to the presence of metal particle(s) in the sample;
wherein the substrate includes an electrically conductive coil printed thereon that circumferentially surrounds said aperture for receiving the sample, the electrically conductive coil forming part of said sensing means; the method comprising locating a sample of a substance to be tested such that it is received in said aperture;
using the magnetic field generating means to generate a magnetic field;
and using the sensing means to sense the result of interaction between the sample and the applied magnetic field.

In any of these further aspects of the invention, the sensed result may be used in any of the manners described herein to obtain information relating to the presence of metal particles within the sample and/or the obtained information may be of any of the types described herein.

In accordance with these further aspects of the invention the method comprises the step of operating the sensor apparatus to cause the magnetic field generating means to generate the magnetic field, and the sensing means to sense the result of the interaction. The magnetic field generating means may be energised to generate the magnetic field once the sample is provided in the aperture, or may be energised before the sample is provided in the aperture. The sensing means may define a sensing region, the sensing means being arranged to sense the result of interaction between the magnetic field and the sample within the sensing region.

The present invention in any of the further aspects may include any or all of the features described in relation to any of the other aspects of the invention to the extent they are not mutually inconsistent. In particular the sensor apparatus may be in accordance with any of the embodiments described herein, and may form part of a sensor system or unit as described herein.

The sensor apparatus of the present invention in any of its aspects or embodiments (other than certain embodiments of the further aspects concerning a sensor apparatus described below for determining a count of particles in a dynamic sample with respect to volume, although even in these further aspects of the invention a sensing coil printed on a substrate is used) comprises a PCB substrate having one or more electrically conductive coils printed thereon. For ease of reference the coils may be referred to as the printed "conductive coils" or the "coils" for brevity herein. The one or more conductive coils include at least the conductive coil that surrounds the aperture for receiving the sample of the substance to be tested. The substrate may have one or more additional conductive coils printed thereon as described below, or may include only one conductive coil printed thereon, being the coil forming part of the sensing means. One or more additional coils may be provided, for example, reference coil(s) as described below. Any of the features described below in relation to a printed coil may be applied to at least the printed coil that surrounds the aperture and forms part of the sensing means, and, where one or more additional printed coil is provided on the substrate, one or more, or preferably each such coil may also comprise any of the features described, unless inconsistent or otherwise stated.

It will be appreciated that any of the features described herein, relating to the printed coils, substrate, and aperture therein of the earlier aspects of the invention, are applicable to preferred embodiments of the further aspects relating to a sensor apparatus for determining a count of particles with respect to volume, which may not, but preferably do, include a sensing coil that is printed on a substrate surrounding an aperture as in the earlier aspects of the invention in any of their embodiments. The sensor apparatus of these further aspects may use sensing means in accordance with the earlier aspects of the invention in any of their embodiments.

In accordance with the invention, the sensor apparatus comprises a substrate comprising the aperture which receives the sample of the substance to be tested, and having the one or more conductive coils printed thereon, including the coil of the sensing means which surrounds the aperture. It will be appreciated that the or each printed coil is therefore not a mechanically wound coil. This is distinct from the coil arrangements used e.g. in US 6,051,970. Preferably each conductive coil of the sensor apparatus is a printed coil, preferably printed on the substrate.

The substrate comprises one or more substrate layer. Thus the substrate may be a single layer substrate or may comprise multiple substrate layers. Where the substrate comprises multiple substrate layers, the substrate layers are joined e.g. laminated together to provide a unitary substrate. A multilayer substrate is preferably of a laminate construction. Laminate multilayer substrates may be produced by separately forming and then joining layers, or by forming one or more additional layer on a base layer.

Whether it is single layer or multilayered, the substrate defines a first side and a second side, with the aperture connecting the first and second sides thereof. The substrate has a first surface and an opposed second surface on the respective first and second sides thereof. The first and second surfaces are the major surfaces of the substrate. The first and second surfaces or sides of the substrate are separated by the thickness of the substrate. Thus the thickness dimension of the substrate extends perpendicular to the first and second surfaces. It will be appreciated that the thickness of a substrate including multiple layers will be a total thickness taking into account each of the layers present. The first and second surfaces are preferably each substantially flat. The surface of the substrate then provides a substantially flat surface upon which components are deposited e.g. a coil or coil portion and, where appropriate, any additional circuitry. The first and second surfaces preferably lie in parallel planes.

The substrate comprises at least one layer. Each layer has first and second sides facing toward the first and second sides of the substrate respectively. Where the substrate is a single layer substrate, the first and second sides of the layer may correspond to the first and second sides of the substrate. Each layer defines first and second opposed surfaces i.e. major surfaces on the first and second sides thereof. Where the substrate is a single layer substrate, the first and second surfaces of the layer may correspond to the first and second surfaces of the substrate. The first and second surfaces of a layer are separated by the thickness of the layer. Thus the thickness dimension of a layer extends perpendicular to the first and second surfaces thereof. Each surface of a layer is preferably substantially flat. The surface of the layer then provides a substantially flat surface upon which components are deposited e.g. a coil or coil portion and, where appropriate, any additional circuitry. The first and second surfaces of the layer preferably lie in parallel planes.

References to the sides of a layer or substrate herein refer to the first and second sides thereof unless otherwise stated.

Whether the substrate is single layer or multiple layered, each printed coil of the substrate comprises one or more coil portions, each coil portion of a given coil being printed at a respective level of the substrate. Each coil portion of a given coil may be printed on a substrate layer of the substrate at a respective level of the substrate. Where a coil comprises multiple coil portions, the coil portions are printed (e.g. on substrate layers of the substrate) at different respective levels of the substrate. This results in a coil which may be described as a "multi-layer" coil. The level of the substrate refers to a particular level through the thickness of the substrate, on or within the substrate. Where a coil includes multiple coil portions, the coil portions are electrically connected together so that they function as a single coil arrangement. A coil portion as used herein refers to the portion of a coil defined on a given surface of a layer of the substrate at a particular level of the substrate. This portion may be made up of one or more electrically conductive traces.

A multilayer coil or coils may be used with either a single layer or a multiple layer substrate. Conversely, a single layer coil or coils may be used with a single or multiple layer substrate.

It will be appreciated that a coil may comprise, or may even consist of, a coil portion or portions printed (e.g. on a surface or surfaces) at a level to the interior of the substrate i.e. on a surface of a substrate layer that lies between the first and second sides of the substrate. Thus references to a coil being printed on the substrate as used herein encompasses at least a portion, or even the entirety of the coil being printed within the substrate.

Where the substrate is a single layer substrate, the or each coil portion of the or each coil is printed on that layer. Where the substrate comprises multiple layers, a coil may comprise one or more coil portions, printed on any one or ones of the layers thereof. Whether the substrate has one or more layers, a coil may comprise coil portions on one or both sides of one or more layers of the substrate. The sides of a layer refer to the first and second sides thereof.

Where a coil comprises multiple coil portions, the portions are located at different levels of the substrate i.e. at different positions through the thickness of the substrate, and are arranged one above the other. The coil portions may be located on different layers and/or sides of a layer or layers of the substrate one above the other through the thickness of the substrate. As mentioned above, such a coil may be referred to as a multilayer coil. At least the coil surrounding the aperture and forming part of the sensing means may comprise multiple coil portions printed on different respective levels of the substrate. Each portion of the coil of the sensing means circumferentially surrounds the aperture.

The portions of a coil at different levels of the substrate e.g. on different layers and/or sides of a layer or layers of the substrate are electrically connected. Portions of a coil at different levels of the substrate i.e. on different layers and/or different sides of a layer or layers of the substrate may be connected using one or more electrical via i.e. through the plane of the substrate (in the thickness direction). It will be appreciated that where a coil comprises multiple coil portions, these may be disposed on any one or ones of the layer or layers of the substrate, and on either or both of the sides of a layer or layers of the substrate as desired, and suitably connected to one another, to provide coil portions at different levels of the substrate. The portions of a given coil function to provide a single coil arrangement. Each coil portion of a coil comprising multiple portions may be of the same or different construction. For example, the number of turns in the different coil portions may vary. In some embodiments each coil portion is of the same construction i.e. shape, number of turns etc.

It should be appreciated that in accordance with any of the aspects or embodiments of the invention, a coil, or coil portion may be defined by a single turn, and therefore references to "turns" of a coil or coil portion do not imply that a coil need have more than one turn, unless mutually inconsistent. The feature(s) may apply to the or each turn of a coil or coil portion.

In some embodiments at least the coil surrounding the aperture and forming part of the sensing means is defined by a single coil portion. The coil may then be referred to as a single layer coil. Preferably the coil portion is printed on the first or the second side of the substrate e.g. on the first or second surface thereof. This arrangement may be used with a single layer or multiple layered substrate.

It will be appreciated that references to "at least the coil surrounding the aperture and forming part of the sensing means" do not require that any further coil is necessary present on the substrate, but if such a coil or coils is present, it may share the specified feature.

In embodiments in which the substrate is a single layer substrate, at least the coil surrounding the aperture and forming part of the sensing means may comprise a portion or portions printed on one or both of the first and second sides of the substrate.

In embodiments in which the substrate comprises multiple layers, at least the coil surrounding the aperture and forming part of the sensing means may comprise portions printed on more than one layer of the substrate, each coil portion being printed at a different level of the substrate. Such a coil may then be described as a multi-layer coil. Coil portions may be printed on either one or both sides of a given layer. In some embodiments in which the substrate comprises multiple layers, at least the coil surrounding the aperture and forming part of the sensing means comprises coil portions printed on one or both of the first and second sides of the substrate, and optionally one or more coil portion printed on a layer of the substrate between the first and second sides thereof. One or more coil portions may therefore be printed on a surface to an interior of the substrate.

Where a coil portion is printed on a first or second side of a layer or of the substrate, the portion may be printed on the first or second surface thereof.

A coil comprising multiple portions on different layers of a multi-layered substrate may be formed using standard printed circuit board manufacturing techniques where the layers are sequentially patterned and laminated together. The turn(s) on adjacent layers should be such that the magnetic field generated by each turn is in the same direction. A coil comprising multiple portions on different sides of a layer of a multi-layered substrate may be produced using a double sided PCB substrate. It will be appreciated that a coil portion may be provided at any level of a PCB substrate where a conductive layer exists that may be used to provide the conductive coil e.g. by suitable etching thereof.

Whether or not the substrate is multi-layered, preferably at least the coil surrounding the aperture and forming part of the sensing means comprises portions printed on one or both of the first and second surfaces of the substrate. It will be appreciated that the first and second surfaces of the substrate may be provided with a coating e.g. a protective coating, such that the coil portions may not be exposed.

Where the substrate comprises multiple conductive coils printed thereon, one or more, and preferably each, of any additional coils may be located on the substrate in the same manner as the coil surrounding the aperture and forming part of the sensing means e.g. having coil portion or portions disposed on the same layer or layers, or side(s) of a layer or layers thereof, with each coil portion being at a given level of the substrate. Thus each additional coil may be a single layer coil or a multi-layer coil. It is also envisaged that a portion or portions of any one of one or more additional coils may be disposed on a different layer or layers or side(s) of a layer or layers of the substrate. In other words, the coil portions of different coils may be located at the same or different levels of the substrate. Preferably each printed coil of the substrate comprises coil portions printed at the same levels of the substrate. Thus, any of the statements applying to "at least the coil surrounding the aperture" above, may apply to the or an, and preferably each additional coil of the substrate if present. In some embodiments the substrate comprises at least one coil printed thereon which is identical to the coil forming part of the sensing means. Such a coil will comprise coil portions located at a corresponding level or levels of the substrate.

In any of the embodiments of the invention, each coil portion of at least the coil surrounding the aperture and forming part of the sensing means, and of each coil printed on the substrate, is printed on a substantially flat surface of a layer of the substrate. The surface of the substrate provides a base upon which the or each coil, and optionally other components are disposed. It will be appreciated that the component or components disposed on the surface may extend from the surface, though typically only by a small distance. The one or more turns of the or each coil portion of at least the coil surrounding the aperture and forming part of the sensing means, and preferably of each coil printed on the substrate, are substantially planar. In contrast, in a solenoidal coil the windings are three dimensional. References to the windings or turn(s) of a printed coil of the present invention of course refer to printed windings or turns.

Preferably each coil portion of at least the coil surrounding the aperture and forming part of the sensing means is a spiral coil portion. A spiral coil portion comprises multiple turns of increasing radius. The turns are coaxial. Each turn is about an axis extending through a radial centre of the coil. The turns are preferably defined in a single plane. Where the coil comprises multiple portions at different levels of the substrate, the resulting coil may be described as a multilayer spiral coil. A coil made up of spiral coil portion(s) may be referred to as a spiral coil i.e. a single or multilayer spiral coil. Where the substrate comprises one or more additional conductive coil comprising one or more coil portions printed thereon, each coil portion of the or each additional coil is preferably a spiral coil portion. A spiral coil portion, or spiral coil (whether single or multilayered), as referred to herein, (whether of or being the coil surrounding the aperture or any other coil), may be circular or non-circular in shape. For example the spiral coil portion or spiral coil may be square, circular, rectangular, oval, racetrack, or triangular in shape, or indeed may be of any other desired shape. Each turn of the coil or coil portion may thus define a substantially square, circular, rectangular, oval, racetrack or triangular shape, or other shape as desired. In some preferred embodiments the or each spiral coil or coil is circular in shape. It will be appreciated that because the coils are printed, it is relatively easy to fabricate a coil of any desired 2D shape. It will be appreciated that a spiral coil or coil portion is in contrast to a toroidal coil or coil portion, which includes turns around an axis extending around the circumference of the coil or coil portion. The or each coil portion of at least the coil surrounding the aperture and forming part of the sensing means, and preferably of each printed coil of the substrate is preferably not a toroidal coil portion.

In general, whether or not it is spiral, a coil or coil portion may be defined by a plurality of coil turns. Each turn may be circular or non-circular, and of any of the shapes previously discussed in relation to spiral coils/coil portions. In other embodiments, a coil or coil portion may be defined by a single coil turn. Such a turn may be circular or non-circular, and of any of the shapes previously discussed in relation to spiral coils/coil portions.

In accordance with the invention in any of its aspects or embodiments, the substrate is in the form of a board. The substrate is a substrate of a printed circuit board (PCB). The or each conductive coil printed thereon forms part of the circuitry of the printed circuit board. The term "printed circuit board (PCB)" as used herein has its usual meaning in the art. The substrate may be substantially planar.

In general, the or each printed coil or coil portion is preferably a coil or coil portion of a PCB.

The thickness of the substrate is may be less than 5 mm, or less than 3 mm. The thickness of the substrate may be at least 0.5 mm, or at least 1 mm. The thickness of the substrate may be between 1 and 2 mm, for instance about 1.6 mm. It will be appreciated that these ranges of thickness may suitably be obtained using standard multi-layer printed circuit board substrates, though single layer substrates may also be used. It will also be understood that the substrate thickness may be greater or less than these values, for example depending upon the material used to form the substrate, the desired rigidity and/or the desired number of layers. The above ranges of thickness are particularly common standard thicknesses for printed circuit boards that use a glass-reinforced plastic substrate, although may be used for other types of substrate. The substrate is preferably of uniform thickness. Where the substrate is of non-uniform thickness, the maximum thickness is less than the upper limits of the ranges defined, and the minimum thickness is greater than the lower limits of the ranges defined.

The substrate may be of any suitable material or materials. The material used for the substrate should be chosen so that it does not significantly deform under normal operating temperatures, pressures or mechanical forces for the particular application in which it is intended to be used. The substrate may be a substantially rigid substrate. In general, any material used in the construction of PCBs may be used. The substrate may comprise or be formed from any one of; glass-reinforced plastic material, plastic material or ceramic material. This list is merely exemplary, and is not limiting. The substrate may comprise or be formed from an epoxy resin, polyimide, or PTFE (polytetrafluoroethylene) material. The skilled person will appreciate that a suitable substrate material may be selected taking into account e.g. desired thermal expansion properties (i.e. how high a degree of thermal stability/low a coefficient of thermal expansion is required), and other desired attributes. Glass reinforced plastic materials may provide suitable thermal expansion properties for many contexts, and are commonplace. The substrate may comprise or be formed from thick or thin film ceramic materials. Ceramic materials, in particular, may be associated with low thermal coefficients of expansion, and may be advantageous for use in providing a substrate for certain applications where a high degree of thermal stability is important. Such materials may readily be used in single substrate layer or multi substrate layer arrangements.

The or each layer of the substrate upon which a coil is printed may be made of or comprise any one of these materials. A given substrate layer may comprise more than one type of material. Where a substrate comprises multiple substrate layers, the layers may be of the same or different materials. The substrate may comprise additional materials as appropriate to laminate the layers thereof together e.g. adhesive layers, and/or may comprise any desired surface coating or coatings. As known in the art, the substrate, or a substrate layer thereof, will provide an electrically insulating layer upon which conductive material is provided in a given pattern i.e. by appropriate printing, to provide the circuitry of the board. This may be achieved by adding conductive material to the surface of the layer, or removing material from a conductive layer deposited thereon.

According to the independent claims, the substrate is a substrate of a PCB, the PCB may be a multilayer or a single layer PCB. Preferably the PCB is multilayer. The term "multilayer" or "single layer" PCB then takes its usual meaning in the art, referring to a PCB having more than one, or a single conductive e.g. foil layer. A single layer PCB may be single sided or double sided i.e. having a conductive e.g. foil layer on one or both sides thereof. A coil printed on the substrate i.e. the coil surrounding the aperture, and optionally any additional coil, may then comprise one or more coil portions, each coil portion being provided at a given one of the conductive layers of the PCB. A multilayer coil will comprise coil portions at different ones of the conductive layers of the PCB, and thus at different levels of the PCB. A conductive layer may be defined by the coil portion, or may comprise additional circuitry.

In some exemplary embodiments, for at least the coil surrounding the aperture, and in embodiments for any or each of the coils printed on the substrate where multiple coils are printed thereon, the ratio between the total width of the coil (measured between two diametrically opposed points on the outermost radial turn of the coil) and the length of the coil (measured perpendicular to the plane of the substrate in the thickness direction of the substrate) may be in the range of; from 5 to 50, from 5 to 25, from 10 to 25, from 10 to 20, from 12 to 20, or from 15 to 20. The length of the coil takes into account all coil portions of the coil at different levels of the substrate when the coil is multi-layered. Because the axial extent of the coil is limited by the thickness of the substrate, it will be understood that the coils described herein are typically relatively short. The length of the coil is the length along its axis i.e. in the direction of the thickness of the substrate between the first and second sides thereof. The width will be the maximum width of the coil (e.g. of a widest coil portion) where a coil or coil portion is of variable width. This may be applicable to circular or non-circular coils.

In accordance with the invention in any of its aspects or embodiments, in some exemplary embodiments, for at least the coil surrounding the aperture, and optionally any or each of the coils printed on the substrate where multiple such coils are provided, the ratio between the outer diameter of the coil (or a coil portion of a coil) and the inner diameter of the coil (or coil portion) may be less than about 1.5, or less than about 1.4, 1.3, 1.2 or 1.1. The outer diameter is the diameter of the radially outermost turn of the coil, or coil portion. The inner diameter is the diameter of the radially innermost turn of the coil, or coil portion. Each coil portion of a given coil may have a ratio of outer to inner diameter within this range. This range is applicable to both circular and non-circular coils. Where a coil includes one or more turns of non-circular shape, the outer or inner diameter will be the maximum diameter or maximum width between two diametrically opposed points of the relevant turn. This relationship may apply to the or each coil portion of a coil, and/or to the overall coil.

The or each conductive coil printed on the layer or layers of the substrate i.e. the or each coil portion or portions thereof) may be printed onto the substrate using any known printing technique. Printing refers to providing a pattern of conductive material on a layer or layers of the substrate defining the shape of the coil. Thus each printed coil is defined by a printed pattern of conductive material. The printed coil or coil portion is in a fixed position with respect to the substrate. The one or more turns of each coil portion of a coil are printed. This is achieved by using a suitable printing pattern to provide the coil turn(s).

In general a coil (i.e. the or each coil portion thereof) may be printed using an additive, a subtractive, or semi-additive printing process. Any current or future technique may be used. For example, it is envisaged that a 3D printing technique might be used. Where multiple coils are provided, they may be provided by the same or different techniques. The following techniques may therefore be used in providing any one or ones of the coils i.e. to providing the or each coil portion thereof.

In general a coil or coil portion is printed on the surface of the substrate, or a layer thereof, by adding conductive material to the surface, or removing conductive material from a conductive layer provided on the surface, or combinations thereof, to result in a pattern of conductive material being provided on the surface of the substrate layer defining the coil or coil portion. By way of example only, the coil or coil portion may be printed using any one or ones of the following techniques; etching, screen printing, ink jet printing, photoengraving, silk screen printing, milling, electroplating. These processes are merely exemplary.

In general any technique known for use in providing a conductive coil on a PCB may be used. Particularly, although not exclusively, where the substrate is a substrate of a printed circuit board, a conductive coil may be printed thereon (e.g. on the surface of layer thereof) by etching a conductive e.g. copper layer provided on the substrate (e.g. on a layer of the substrate). A coil or coil portion may alternatively be formed by directly printing conductive ink onto the substrate. The conductive ink may contain flakes of conductive material such as powdered copper, silver or carbon or may be of an inherently conductive material, e.g. a conductive polymer or other conductive organic material, such as graphene. Any of the above printing techniques may be highly reproducible from coil to coil, or product to product. Each coil portion of a multi portion coil may be provided in any of these manners. It will be understood that printing processes are distinct from mechanical winding processes as used e.g. in US 6,051,970. The sensor apparatus may be free from mechanically wound coils. The or each printed coil or coil portion does not include a wound solid wire.

In some embodiments the or each printed coil, or coil portion thereof, is printed using a technique involving one or both of; etching to remove conductive material from a conductive layer (e.g. on a surface or surface) to leave a pattern of conductive material defining the coil or coil portion (e.g. on the or each surface), or adding a conductive material (e.g. to a surface or surfaces) in a pattern defining the coil or coil portion, or combinations thereof.

It may be seen that the or each coil portion of a printed coil will be substantially flat or 2 dimensional. The or each portion of the coil may be described as substantially flat or 2 dimensional. Rather than referring to a coil portion of a coil of the apparatus as being printed at any point herein, a coil portion may be referred to as substantially flat, or 2-dimensional. Thus a printed coil may instead be referred to as a coil having one or more substantially flat coil portions, each provided at a given level of the substrate.

In accordance with a further aspect of the invention there is provided a sensor apparatus for use in obtaining information relating to the presence of metal particles within a sample of a substance to be tested, the apparatus comprising:
a PCB substrate comprising an aperture for receiving a sample of a substance to be tested, the substrate having one or more electrically conductive coils thereon;
magnetic field generating means for generating a magnetic field for application to a sample received in the aperture in use; and
sensing means for sensing the result of interaction between the generated magnetic field and a sample received in the aperture, the sensed result of the interaction being usable to determine information relating to the presence of metal particle(s) in the sample;
wherein the substrate includes an electrically conductive coil thereon that circumferentially surrounds said aperture for receiving the sample, the electrically conductive coil forming part of said sensing means,
wherein the or each coil of the substrate comprises one or more coil portions, each coil portion of a given coil being provided at a respective level of the substrate, wherein each coil portion is substantially flat. Each coil portion may be provided on a substrate layer of the substrate at a given level of the substrate.

The present invention in this further aspect may include any or all of the features described in relation to the other aspects and embodiments of the invention, to the extent they are not mutually exclusive. Any of the further aspects of the invention e.g. methods etc. may refer to a coil being made up of one or more substantially flat coil portions, rather than to the coil being a printed coil.

In accordance with the invention in any of its aspects or embodiments, the substrate comprises an aperture therethrough for receiving a sample of the substance to be tested. The aperture extends completely through the substrate from a first side to a second side thereof. Preferably, the aperture for receiving the sample is defined solely by the substrate having the one or more conductive coils. The sample is received in the aperture in that at least a portion of the sample is located within the aperture. The sample may extend beyond the aperture on one or both sides thereof. An exemplary substrate for use with any of the embodiments or features described herein is a printed circuit board. The sensor apparatus may thus comprise a printed circuit board defining the substrate and comprising at least the aperture, the sensing means and optionally the magnetic field generating means.

The coil surrounding the aperture forms part of the sensing means for sensing the result of interaction between the sample and the generated magnetic field. The coil is preferably the only coil of the sensing means. Any additional coil of the sensing means is preferably a printed coil, preferably provided on the substrate, and which may be of the same construction.

The sensing means has a sensing region. In some preferred embodiments the sensing region is defined by the coil surrounding the aperture which forms part of the sensing means. The sensing region comprises at least a portion, and may include the entire area and/or volume defined by the aperture. The sensing region may extend in an axial direction through the aperture i.e. in the direction of the axis of the coil/the thickness of the substrate. The sensing region may extend any given distance on one or both sides of the aperture, although is preferably relatively short in the axial direction. The extent of the sensing region will typically depend upon the coil arrangement, as discussed herein. The extent of the sensing region may also be influenced by the nature of the substance being tested.

The sensing means is arranged to sense the result of interaction e.g. within the sensing region between the generated magnetic field and the sample received in the aperture. Thus such interaction may be sensed when it occurs within the sensing region. For extracted samples, at least a portion of the sample will be located within a sensing region of the sensing means. The sample may or may not extend through the entire sensing region in the axial direction and/or extend beyond the sensing region in the axial direction. Where the sample is a dynamic sample, the sample will typically correspond to the volume of fluid flowing through the aperture between first and second times e.g. of a test period. Such fluid will pass through at least a portion of a sensing region.

Preferably the coil surrounding the aperture provides the sensing means. In these embodiments the sensing means consists of the coil. In other words, the sensing means is defined solely by the printed coil.

The magnetic field generating means may or may not be provided on the same substrate as the coil of the sensing means. The magnetic field generating means may be provided on another substrate, or in any other manner. The magnetic field generating means for generating the magnetic field for application to the sample preferably comprises an electrically conductive coil. The coil is preferably one of the one or more conductive coils printed on the substrate. The coil may or may not be the same coil that surrounds the aperture and forms part of the sensing means. The coil of the magnetic field generating means may comprise one or more coil portions, and may be a single layer or multiple layer coil as previously discussed. The magnetic field generating means may further comprise means for providing a supply of current to the coil. The magnetic field generating means may comprise a single coil. The coil may be a single layer coil or a multiple layer coil comprising portions on different levels of the substrate.

Desirably the same coil is used to generate the applied magnetic field and to sense the result of interaction between the sample and the generated magnetic field. In this case, this coil is preferably the only coil surrounding the aperture. As discussed above, the coil may comprise portions at different levels of the substrate i.e. provided on multiple layers and/or surfaces of a layer or layers of the substrate. Thus the coil may be a multilayer coil. In other embodiments the coil may be a single layer coil comprising a single coil portion printed on the or a layer of the substrate. In preferred embodiments, therefore, the printed conductive coil circumferentially surrounding said aperture that forms part of the sensing means also forms part of said magnetic field generating means. The magnetic field generating means may then comprise the conductive coil circumferentially surrounding the aperture and means for providing a supply of current to said coil.

The coil surrounding the aperture and forming part of the sensing means may be referred to as "the sensing coil" herein.

Preferably the printed coil surrounding the aperture is the only coil of the sensing means and the magnetic field generating means. As mentioned above, for optimum sensitivity, it is desired for the most sensitive part of the sensing means to be located where the applied magnetic field is strongest, i.e. for the sensing coil and the magnetic field generating coil to overlap and to both be as close to the sample as possible. This may be achieved by using a single coil for both magnetic field generating and sensing. This also avoids any risk of relative movement between the sensing and generating means. Using a single coil for magnetic field generation and sensing is particularly suited to printed coils as space on the substrate can be most effectively used without adding excess bulk to the sensor apparatus.

Nonetheless, it is envisaged that separate coils may be used to generate the applied magnetic field and to sense metal particles. The magnetic field generating means might therefore, in alternative embodiments, comprise a conductive coil that is additional to, and distinct from, the coil of the sensing means. The coil may be another coil printed on the or a substrate. Preferably the coil is printed on the same substrate as the coil of the sensing means. The coil of the magnetic field generating means may comprise one or more coil portions, each provided on a level of the substrate. By using separate coils, the coils may be independently optimised for transmitting or receiving. However, since the coils are not co-extensive a compromise has to be made in relation to the position of at least one of the coils relative to the sample. It is also contemplated that an applied magnetic field may be generated in some other way, not necessarily utilising a coil.

The sensing means detects changes in magnetic moment due to the generated magnetic field. Typically, in accordance with the invention in any of its aspects or embodiments, the generated magnetic field is an alternating magnetic field. The magnetic field generating means is arranged to provide an alternating magnetic field in use. This may be achieved by connecting the magnetic field generating means to a power supply arranged to provide an alternating current. In accordance with the invention in any of its embodiments, preferably the method of using the magnetic field generating means to generate a magnetic field comprises supplying an alternating current to the magnetic field generating means in order to generate an alternating magnetic field. The alternating current may be supplied to a coil of the magnetic field generating means e.g. in preferred embodiments to the coil which also forms part of the sensing means, in order to generate an alternating magnetic field. Preferably the magnetic field generating means comprises the coil of the sensing means that surrounds the aperture and means for providing a supply of alternating current thereto. The means for supplying alternating current may be an input connected to or connectable to a source of alternating current. Where a reference component(s) e.g. coil is provided, it preferably shares the same source of alternating current.

The frequency that an alternating current is supplied at may be in the range 1 kHz - 100 MHz. The driving frequency may be selected based on the size of particles to be measured or the type of measurement being performed. It has been found that higher frequencies, for instance in the range 1 MHz to 100 MHz, or 5 MHz to 100 MHz, may be particularly useful in detecting e.g. counting individual particles in a dynamic sample defined by a flow of fluid, as such frequencies may increase the sensitivity of the apparatus. The driving frequency will affect the impedance of the sensing coil, and so a higher frequency may allow a smaller change in inductance to be measured. It has been found that frequencies lower than about 5 MHz, or 1 MHz, may be particularly useful for determining the quantity of ferromagnetic e.g. ferrous material within a sample.

The magnitude of the magnetic field generated by a driven coil will be determined in part by the number of coil turns and in part by the distance from the turns of the coil to its axis. This will in turn determine the ultimate sensitivity of the sensor apparatus. In order to increase sensitivity, it is generally desirable therefore to provide as many turns in the coil as possible, and arrange these turns as close to the sample as possible.

In order to achieve higher sensitivity it is desirable to be able to increase the number of coil turns of the sensing coil. The use of the printed sensing coil of the present invention allows this to be achieved more readily. The number of turns for the printed coils described herein may be increased by choosing appropriate coil geometries, e.g. by printing a single or multi-layered spiral coil with an appropriate number of turns in the or each coil portion. The printing process provides greater flexibility in being able to readily provide a coil of a desired configuration in a cost effective process, simply by modifying the printing pattern, in contrast to prior art techniques which use mechanically wound coils. It has been found that by suitably selecting the number of turns of the sensing coil, the sensitivity of the sensor apparatus can be increased to comparable levels to that of a conventional solenoidal coil should this be desired for a given application.

In accordance with the invention in any of its aspects or embodiments, the sensing coil may comprise any number of turns in the or each coil portion, but it will be understood that the turns contribute less and less to the measurement with distance radially from the centre of the coil or coil portion, as they become further from the centre of the aperture. For instance, the coil may comprise one or more, e.g. a plurality of turns in the or each coil portion, and particularly may comprise at least 6 turns in each coil portion. The coil may comprise no more than 12, or no more than 10 turns in each coil portion. In some exemplary embodiments the coil comprises 8 turns in each coil portion. To minimise any loss in sensitivity, it is desirable that the coil should not be excessively wide compared to the sample, i.e. the coil turns should be spaced as close to each other, and to the edge of the aperture, as practical. In certain applications, such as the particle counting application described herein, it may be advantageous to ensure that the magnetic field experienced by the particles is uniform, otherwise particles passing a stronger region of the field may appear bigger. In this case, the size of the aperture may be selected to be slightly smaller than the internal diameter of the coil. It will be appreciated that there is a trade-off between having the coil as close to the sample as practical and having the field be as uniform as possible. This may be optimised for a particular application by controlling the relative sizes e.g. diameters of the coil and aperture. Where a coil comprises multiple portions, the portions may comprise the same or differing numbers of turns, preferably within the above ranges.

For an elongate solenoidal coil it is relatively simple to have a large number of turns close to the axis by increasing the number of turns of the coil, and hence the coil length. This may be adequate for measuring the total particle content in an elongate static sample. However, the applicant has realised that when detecting/counting individual particles that are of a dimension much smaller than the length of the coil, e.g. in a dynamic sample defined by a flow of fluid, most of the turns of an elongate solenoidal coil would be too far away from a particle passing along the coil axis at any given time to contribute significantly to the measurement. The applicant has realised that in the context of detecting/counting individual particles in a dynamic sample defined by a flow of fluid, it would be particularly desirable to use a coil that is relatively short in the direction of flow, or even planar, so as to reduce the size of the sensing volume and hence the level of background noise. As described below, background noise may arise from the presence of particles that are too small to be detected individually, but provide an aggregate signal that constitutes background noise The use of the printed sensing coil of the present invention is therefore particularly advantageous in this context, enabling the length of the coil to be minimised in the direction of flow i.e. in the axial direction of the coil. In accordance with the invention, the coil of the sensing means is relatively short in the axial direction, and is planar. Even where the coil is a multilayer coil, the substrate may be relatively thin, resulting in a coil of short length.

In accordance with the invention in any of its aspects or embodiments the aperture is preferably substantially circular. However, this is not necessarily the case, and non-circular shapes may be used. For example, the shape of the aperture may match the shape of the turn(s) of the coil of the sensing means surrounding the aperture.

The aperture defines a space within which a sample of the substance to be tested is received. The aperture defines a radial direction and an axial direction. The axial direction of the aperture corresponds to the direction of the thickness of the substrate. This direction is generally referred to as the axial direction herein. Preferably the axial extent of the aperture is defined by the thickness of the substrate. The aperture extends between and connects the first and second sides of the substrate. The axial direction may extend perpendicular to planes in which the first and second surfaces of the substrate lie. As the substrate is desirably thin, the aperture may have minimal extent in the axial direction, and may be substantially planar. It will be understood that the sample will generally extend through the aperture, and extend axially beyond the thickness of the substrate. The sample may extend at least axially throughout a sensing region, and may extend axially beyond the sensing region.

The coil forming part of the sensing means completely circumferentially surrounds the aperture. The coil forming part of the sensing means is preferably coaxial with the aperture. The coil is preferably located adjacent the periphery of the aperture. As discussed above, the coil may comprise multiple coil portions at different levels of the substrate. Each coil portion is then coaxial with the aperture and circumferentially surrounds the aperture at a different level of the substrate. The coil, or each portion thereof, is desirably printed as close to the aperture as practical. Where the coil comprises multiple portions, each portion is preferably located adjacent the periphery of the aperture.

In accordance with the invention in any of its aspects the sample to be tested may be a sample of any substance desired to be tested to obtain information relating to the presence of metal particles therein. The apparatus may therefore be configured for testing any such substance. Preferably the sample is a fluid sample. In accordance with the invention in any of its aspects or embodiments in which the sample is a fluid sample, including the further aspects which may not use printed/flat coils, the fluid is preferably a liquid. In the absence of metal particles the sample fluid will typically be non-electrically conductive and non-magnetic, or only weakly electrically conductive or magnetic so that a signal indicative of the result of interaction between the sample and the magnetic field is essentially zero. The fluid may comprise or be oil, grease or water, or any combination thereof. The fluid may be of any desired degree of viscosity, and may include relatively high viscosity fluids such as grease. The present invention is particularly applicable to testing a sample of a fluid in the form of a lubricating oil or grease from an engine, turbine or gearbox. In this case any metal particles detected may constitute metal wear debris. It is envisaged that the present invention may also be applied to testing samples of solid substances, such as a mixture of solid particles, or a conglomerate solid material. In these cases the sensor apparatus could be used to determine the total quantity of ferromagnetic particles in the material.

In preferred embodiments the sample is a sample of a lubricant grease or oil from an engine, turbine or gearbox. A turbine may be e.g. a gas turbine, air turbine, or a wind turbine. In particularly preferred embodiments, whatever its nature, the sample is a sample of fluid derived from a marine engine. It will be understood however that the sensor apparatus and techniques described herein are not limited to such applications, and may also be applied more generally to determining information relating to the presence of any metal particles in a sample of any substance e.g. a sample of fluid. For example, the sensor apparatus may be used to test samples of ionic solutions, magnetic particles used in biochemical assays, or drinking water (e.g. to test for contaminants). The sensor apparatus may also find use in oil or gas well operations.

It will be appreciated that in accordance with the further aspects of the invention relating to an apparatus and method for determining a count of particles in a dynamic fluid sample with respect to volume, in which printed coils may or may not be used, the fluid sampled may be of any of the types discussed in relation to possible types of fluid sample, e.g. a liquid such as a lubricating oil or grease. The metal particles detected may be metal wear debris. The sample may be from any of the sources mentioned e.g. an engine, turbine, gearbox etc., although is not limited to such fluids, and the apparatus may be used in testing samples of a wide range of further fluids such as those envisaged above.

The sensor apparatus of the invention in any of its aspects or embodiments may be used to obtain information relating to the presence of metal particles in a substance at different times i.e. to monitor the substance for information relating to presence of metal particles within the substance. This may be particularly appropriate where the sample is a dynamic sample e.g. defined by a flow of fluid.

In some preferred embodiments the sensor apparatus of any of the aspects of the invention may be used in detecting or monitoring wear in a system.

Whatever its nature, the sample of the substance to be tested is, in those aspects and embodiments including a substrate having an aperture surrounded by a printed coil, received within the space defined by the aperture for testing. Thus the aperture defines the measurement axis of the sensor apparatus.

The sample may be a static sample or a dynamic sample. A dynamic sample may be defined by a volume of fluid flowing through the aperture between first and second times. The first and second times define a test period. The first and second times may be any reference times. It will be appreciated that the first and second times may or may not be predetermined times to define a predetermined test period. The apparatus may be used to provide testing in one or more discrete test period, or may be used to provide testing continually. Thus the second time may be a current time or a past time. The first time may be any past time i.e. earlier than the second time. A test period may refer to any period between first and second times, whether or not it is predefined, and includes a period defined between e.g. a past time and current time during continual monitoring.

The sample may be an extracted sample of the substance. In some embodiments the sample is located in a sample container, such as a test tube. The step of locating the sample such that it is received in the aperture may comprise locating a sample container containing a sample of the substance to be tested in the aperture e.g. inserting the sample container through the aperture. The aperture may therefore be configured to receive a sample container containing a sample of a substance to be tested e.g. a test tube. In these embodiments the sample may be a static sample e.g. of fluid. The sample may be of a fluid or a solid. Where the sample of the substance to be tested is located within a sample container, the sample container is preferably located so as to be coaxial with the aperture for sample testing. In these embodiments the sample will typically extend through the aperture, and may extend axially beyond the aperture in one or both directions. This will result in at least a portion of the sample being located in a sensing region. The sample may extend at least axially throughout the sensing region.

However, it will be appreciated that the sample need not be an extracted sample. In other embodiments the sensor apparatus is used to test a sample in situ. In situ testing enables a substance to be tested within a system in which it is used, without needing to extract a sample. The sensor apparatus may be used to test a sample in an in-line test.

In any of the embodiments, whether the sample is an extracted sample or not, where the sample is a fluid, the sample may be a dynamic sample defined by a volume of fluid flowing through the aperture in between first and second times, or may be a static fluid sample. For a dynamic sample, the volume of fluid flowing through the aperture may correspond to the volume of fluid flowing through the sensing region. Dynamic samples are particularly applicable to in situ testing. In some embodiments the sensor apparatus is -located or configured to be located e.g. mounted with respect to a system in which the substance to be tested is used in a manner such that a sample to be tested may be received in the aperture to enable a sample of the substance to be tested in situ. The method may comprise locating e.g. mounting the apparatus with respect to a system in which the substance to be tested is used in a manner such that the sample to be tested is received in the aperture to enable the sample to be tested in situ. The apparatus may be mounted such that a fluid to be tested flows through the aperture to provide a dynamic sample. The apparatus may be mounted in any suitable manner with respect to a path along which the fluid flows. The fluid may be any process fluid. Thus, in some embodiments, at least a portion of a flow of process fluid, and preferably only a portion thereof, flows through the aperture. That is, in embodiments, a portion of the process fluid is diverted into or otherwise sampled by the aperture. The system in which the fluid is used may be, e.g. an engine, such as a marine engine, although the invention is applicable to other contexts as described above. The sensor apparatus may be located e.g. mounted in any suitable manner with respect to a part or parts of the system such that the aperture receives a sample of the substance to be tested in use. These embodiments are particularly applicable to fluid samples. In some embodiments the method may comprise locating the sensor apparatus with respect to the system such that a portion of the substrate defining the aperture with the sensing coil therearound is immersed in a fluid to be tested. In any of these embodiments the fluid may be a flowing fluid providing a dynamic sample.

The sensor apparatus may be used for a one off test e.g. by locating the apparatus with respect to the system so as to receive a sample of the substance. Thus the sensor apparatus may be removed from the system after the sample has been tested i.e. after the result of interaction between the field and the substance has been sensed. For example, the apparatus, or a part thereof, might be dipped into a fluid reservoir, or may be used to test a dynamic sample over a single predefined test period. However, preferably the sensor apparatus is mounted with respect to the system to permit testing of samples of the substance on multiple occasions at different times. The apparatus may be permanently mounted with respect to the system. In other words, different samples will be tested at different times. For example, the sensor apparatus may be arranged to monitor the substance e.g. continually. The method may comprise using the apparatus to monitor a substance for information relating to the presence of metal particles therein. In any of its embodiments, it is envisaged that the apparatus may be retrofitted to a given system e.g. pipe.

Where it is mentioned that the apparatus may be mounted in a given manner herein, the invention also extends to the apparatus configured to be mounted in such a manner.

In some embodiments in which the sample is a fluid sample, the sensor apparatus is mounted or configured to be mounted with respect to a fluid pipe of the system such that fluid flowing along the pipe flows through the aperture to provide a dynamic sample of the fluid. A fluid pipe may be any fluid conduit along which fluid flows, and may be of any diameter. The pipe may be a main flow pipe of the system, or may be a pipe which has been introduced specifically for the purposes of testing the fluid in the system e.g. as part of a bypass It will be appreciated that this is the case in any of the aspects or embodiments of the invention in which a sensor apparatus is mounted to a fluid pipe, including those further aspects of the invention relating to an apparatus for determining a count of particles with respect to volume, whether or not printed coils are utilised.

A dynamic sample is defined by the volume of a substance flowing through the aperture of the apparatus within a given test period. A test period is a period between first and second times, which may or may not be predefined, as described herein. The apparatus may be arranged to continually monitor a flowing fluid, or may be activated for one or more discrete given periods.

The sensor apparatus may be mounted in any suitable manner with respect to a fluid pipe so that fluid flowing along the pipe flows through the aperture to provide the sample. The fluid thus flows through the sensing region. The sensor apparatus may be mounted around the pipe, or may be mounted between sections of the pipe and/or at an end of the pipe. The sensor apparatus may be mounted such that a portion of the substrate defining the aperture with the sensing coil therearound is located within the pipe. The portion of the substrate with the aperture and coil may then be directly within the path of the fluid. The portion of the substrate having the conductive coil of the sensing means and the aperture may therefore be immersed, or configured to be immersed, in the fluid. However, it will be appreciated that the coil need not be immersed, or configured to be immersed, in the fluid, provided that fluid flows through the aperture and hence through the sensing region. In embodiments, therefore, the sensor apparatus may be mounted or mountable such that at least the aperture is within the path of fluid. A portion of the substrate surrounding the aperture may also be immersed in fluid. In some embodiments the sensing coil surrounding the aperture may then also be immersed. However, this need not necessarily be the case. For example, a housing of a sensor apparatus may be configured such that fluid may flow through the aperture, contacting the substrate around the aperture, but without contacting the coil. The fluid may only contact portions of the substrate between the first and second sides thereof which define the walls of the aperture, and not the surfaces in the planes of the first or second sides of the substrate. In other embodiments, the substrate may not contact fluid. For example, a sensor unit comprising the sensor apparatus may comprise a passage defined by a tube extending through the aperture through which fluid flows in order to flow through the aperture without contacting the substrate.

-In some embodiments in which the sensor apparatus is mounted with respect to a system so that fluid from the system flows through the aperture to provide a sample, whether or not the sensor apparatus is mounted or mountable with respect to a fluid pipe of the system, the sensor apparatus is mounted such that a portion of the substrate surrounding the aperture is immersed in the fluid so as to contact the fluid (or will be in use). The sensing coil may also be immersed in the fluid so as to contact the fluid (or will be in use). In other embodiments the sensor apparatus may be mounted such that fluid flows through the aperture without contacting the substrate i.e. such that the substrate is not immersed in fluid (in use). The apparatus may be mounted or mountable so that only a portion of the substrate is (or will be) immersed in the fluid so as to contact the fluid, the portion including the aperture and optionally the sensing coil. As discussed below, the sensor apparatus may form part of a sensor unit, having a first portion configured for insertion into the path of the fluid e.g. a probe portion. The portion of the substrate surrounding the aperture and optionally including the sensing coil may then form part of this portion of the unit, and the unit may be configured such that a portion of the substrate surrounding the aperture, and optionally the coil of the sensing means contacts the fluid. In this case, the substrate may define in part a passage through the first portion of the sensor unit through which fluid flows. In some preferred embodiments a flow sensor may also be disposed in the passage as discussed below, and may optionally be provided on the portion of the substrate. Other parts of the sensor apparatus may be located such that they do not contact the fluid. For example, a portion of the substrate having a reference coil may be located such that it does not contact the fluid. A portion of the substrate having a reference coil may be sealed from the fluid flow.

The apparatus may be mountable or mounted such that a portion of the substrate having the aperture, and optionally the sensing coil (and optionally a flow sensor) is arranged to project through an opening in a wall of a fluid pipe for disposing the portion of the substrate in contact with the fluid. The sensor apparatus may be configured to enable it to be mounted in such a manner. The sensor apparatus may comprise a housing configured to enable mounting in this manner. In some embodiments as discussed below, the sensor apparatus forms part of a sensor unit having a first portion configured to be immersed in a fluid to be tested so that fluid passes through the aperture. The first portion may comprise a portion of the substrate having the aperture and sensing coil (and optionally a flow sensor) which is configured to be immersed in a fluid to be tested to immerse the portion of the substrate in fluid. In other embodiments, the first portion of the sensor unit may be arranged such that when immersed in fluid, fluid passes through the aperture (and, where provided, contacts a flow sensor), but without the sensing coil, or optionally the substrate contacting the fluid. The apparatus (or unit) may comprise a second portion not intended to be immersed in the fluid. The second portion may comprise a portion of the substrate including one or more reference components e.g. one or more reference coils.

In preferred embodiments in which the apparatus is mounted with respect to a system in which a fluid to be tested is used in a manner such that the fluid flows through the aperture to provide a dynamic sample, however the apparatus is configured, and whichever part(s) of the apparatus are or are not immersed in fluid, the aperture is configured to receive only a portion i.e. a fraction of the fluid flow. In other words, only a portion of the process flow in the region of the apparatus passes through the aperture. Preferably where the sensor apparatus is mounted with respect to a fluid pipe so that fluid flowing along the pipe flows through the aperture to provide the sample, the sensor apparatus is mounted such that only a portion i.e. a fraction of the fluid flowing through the pipe passes through the aperture. Thus, in these embodiments, the aperture samples only a portion of the fluid flowing through the pipe. That is, the size of the sensor apparatus and particularly the size of the aperture of the sensing coil may be relatively small compared to the diameter of the pipe. It has been found that by providing a relatively small sensor that is arranged to sample only a fraction of a total flow in the region of the sensor, the sensor apparatus may be able to detect smaller metal particles that are in suspension in the fluid, and which may give an early indication of a wear problem. The Applicant has found that certain prior art sensors which are arranged to sample the entire process flow of fluid e.g. through a pipe have the disadvantage that the sensing coil may need to be of such large dimensions in order to accommodate the pipe, that they are unable to detect smaller particles. While the detection of larger particles may be of use in identifying a potential system failure i.e. where wear is so severe that the system will need to be imminently shut down, it is desirable to be able to detect smaller particles, which can give earlier warning of a problem, when it is still possible to take corrective action. Furthermore, larger particles may be less likely to be detected, as they are less likely to remain in suspension in the system fluid. While the present invention may be used to sense the entire flow of fluid through e.g. a pipe, and may use any suitable diameter sensing coil, the use of the printed sensing coil in accordance with the invention enables the dimensions of the coil of the sensing means to be minimised, making it particularly suitable for sensing only a portion of a fluid flow through a system. Printing techniques may more readily allow a coil of any desired dimension to be produced.

The method may comprise locating or mounting the sensor apparatus in any of the above manners. The sensor apparatus may be configured to be mountable in any of the above manners in use. The invention also extends to the apparatus being mounted in any of the above manners.

In some embodiments the sensor apparatus may be mounted around a fluid pipe of a system with the fluid pipe extending through the aperture. The aperture will then surround the pipe. The sensor apparatus may then surround the pipe.

In yet other embodiments the sensor apparatus may be mounted to a downstream end of a first section of a fluid pipe of a system in a manner such that at least a portion of the fluid flowing out of the first section of the pipe flows through the aperture. The sensor apparatus may connect the first portion of pipe to a second portion of pipe downstream of the apparatus or may define a new end to the pipe downstream of the end of the first section of pipe. In these embodiments a portion of the sensor apparatus will form a portion of the pipe, either connecting first and second pipe sections or providing a new end to the pipe. In these embodiments the sensing coil and the aperture may be immersed in the fluid. The apparatus may comprise one or more connector for connecting the apparatus to sections of pipe upstream and/or downstream of the apparatus. The or each connector may be fixed to a remainder of the apparatus, or releaseably attached thereto. The or each connector may be separately formed from and attached to a remainder the apparatus, or may be integrally formed therewith. In some preferred embodiments the connector is a section of pipe. Thus, the apparatus may comprise a section of pipe for connecting the apparatus to sections of pipe upstream and/or downstream of the apparatus. In some embodiments, as described above, the sensor apparatus is mounted with respect to a fluid pipe of a system such that a portion of the substrate surrounding the aperture for receiving the substance to be tested, and optionally including the sensing coil, is immersed in the fluid flowing along the pipe so as to contact the fluid. The portion of the substrate surrounding the aperture, and optionally having the sensing coil, may be arranged to extend through an opening in a wall of the section of pipe to enable the portion of the substrate to be immersed in fluid passing along the pipe section in use. However, it will be appreciated that where a portion of the substrate surrounding the aperture, and optionally including the sensing coil, extends through an opening in a wall of the section of pipe, at least the sensing coil, and optionally the substrate surrounding the aperture need not be immersed in fluid passing along the pipe in use. The portion of the substrate may be sealed from the contact with the fluid provided that fluid may pass through the aperture. Whether or not a portion of the substrate is immersed in fluid, a portion of the apparatus may be disposed on the exterior of the wall of the pipe section so as to be sealed from fluid flowing along the pipe section in use. This portion of the apparatus may be disposed in a housing of the apparatus. As described below, the apparatus may form part of a sensor unit having a first portion extending through the aperture and immersed in the fluid, and a second portion located to the exterior of the section of pipe. The first portion of the sensor unit is configured such that fluid flows through the aperture, and may or may not be configured such that a portion of the substrate is immersed in fluid so as to contact the fluid.

In other embodiments it is envisaged that the apparatus may comprise a fluid conduit extending through the aperture and defining first and second ends, one or both of which may be connected to an end of a section of pipe of a system comprising a fluid to be tested for mounting the apparatus with respect to a pipe in a manner such that fluid flowing along the pipe flows through the aperture to provide the sample. This may avoid the fluid contacting the sensing coil/aperture. Each end of the fluid conduit of the apparatus may then comprise means for connecting the respective end of the conduit to an end of a pipe section. In these embodiments including a fluid conduit, the fluid conduit forms part of the sensor apparatus and is connected e.g. fixed thereto. The fluid conduit may form part of a sensor unit provided by the sensor apparatus. Where the apparatus comprises a housing, each end of the fluid conduit of the apparatus may extend out of the housing of the apparatus for connection to an end of a pipe section of a system.

Of course, the sensor apparatus may alternatively be mounted with respect to any other fluid container of a system using the fluid to be tested to provide a sample of the fluid for testing i.e. within the aperture, and need not be mounted to a fluid pipe of the system. The apparatus may then be mounted e.g. around, between parts of, or to such a container in any of the manners described with respect to the fluid pipe embodiments to provide a sample of fluid. The sample of fluid will typically be a dynamic sample. In general, the sensor apparatus may be mounted or configured to mountable to any part of a system using a fluid to be tested that contains the fluid in use to provide the sample of fluid for testing, and references to a "pipe" herein may be replaced by a reference to a "part" of the system.

Whether used for in situ or extracted sample testing, or for dynamic or static sample testing, it will be seen that the sensor apparatus may be mounted such that a portion of the substrate comprising the sensing coil is immersed in a sample of a substance to be tested, or may be separated from the sample by the walls of a sample container or of a fluid conduit or pipe extending through the aperture, of the apparatus or of a system including the substance to be tested. It may be advantageous for the conductive coil of the sensing means to be immersed in sample fluid. In this way the coil turns can be situated even closer to the sample being measured. Thus, in some embodiments a portion of the substrate defining the aperture with the sensing coil therearound is immersed in a fluid to be tested. Engine oil may be relatively hot, so sample containers or parts of a system e.g. flow pipes containing engine oil must be able to withstand high temperatures and pressures and may require relatively thick walls. If the sensing coil is mounted within a flow pipe or other fluid container, this eliminates the presence of the walls between the coil and the sample, enabling the coil turns to be situated closer to the sample fluid by at least the wall thickness. Where the conductive coil is immersed in the sample fluid, the coil may be covered by electrically insulating material to prevent electrical shorting. Wired or wireless connections may be provided between the conductive coil and external circuitry e.g. for transmitting data indicative of the result of the interaction between the sample and the magnetic field. Wired connections may avoid any problem with attenuation by a metal wall e.g. pipe.

In accordance with the invention in any of its aspects or embodiments, the diameter of the aperture will depend on the context in which the apparatus is to be used e.g. depending upon the diameter of a sample container or portion of a system e.g. flow pipe or a fraction of a flow to be accommodated.

Desirably, the magnetic field at the centre of the aperture is substantially parallel to a thickness dimension of the substrate. This is the axial direction with respect to the aperture or coil. The magnetic field at the centre of the aperture may be substantially perpendicular to the plane of the first and second surfaces of the substrate. The magnetic field strength may be largest at or near to the centre of the aperture. The field strength should be relatively high where the sensing means is most sensitive. The magnetic field generally permeates the sensor apparatus, with its strength decreasing with distance from the magnetic field generating means.

The sensing means defines a sensing region e.g. volume wherein metal particles within the sensing region contribute to the sensed interaction and metal particles outside of the sensing region do not. The sensing means may then sense interaction between metal particles in a sample, or portion thereof which lies in the sensing region. It will be understood that the extent of the sensing region is determined by the sensitivity of the coil that forms the sensing means. The extent of the sensing region may also depend upon the nature of the substance providing the sample, or any metal particles therein. For example, certain types of metal particle may be more easily detectable than others, meaning that the sensing region may be of greater extent for samples containing such particles. It will also be understood that where the sample is a static or extracted sample of a substance the sensing region of a particular coil may be predetermined for that particular substance. Where the sample is a static or extracted sample, the sensor apparatus may be calibrated using one or more samples of a similar material e.g. containing a known quantity of iron (for instance). Because the magnetic field decreases axially with distance away from the coil the sensing region may be relatively localised axially around the aperture. To enable accurate comparisons between measurements, and with the calibration data, the sample should advantageously extend throughout, and preferably beyond, the sensing region, at least in an axial direction, so that the sensing means always senses an interaction resulting from the same amount of sample i.e. that located within the sensing region. Where the sample is a dynamic sample it will be appreciated that the extent of the sensing region may vary dynamically, in that the substance being tested may dynamically vary, depending upon the type of particles that happen to flow through, or into vicinity with the aperture. The type of calibration discussed above will therefore typically not be appropriate for dynamic sample arrangements.

It is well known how the result of interaction between a sample and a generated magnetic field may be used to obtain information relating to the presence of metal particles within the sample. All metals are electrically conductive, and some metals also exhibit some form of magnetic behaviour. For instance, metals having a spontaneous net magnetic moment, such as iron, nickel and cobalt, are classified as ferromagnetic. Ferromagnetic materials may be classified as "soft" or "hard". Soft ferromagnets have a low coercivity, i.e. the net magnetisation can easily be reversed e.g. by changing the magnetic field direction. Hard ferromagnets on the other hand tend to retain their net magnetisation even under the influence of an applied opposing field. In embodiments, the sensor apparatus of the present invention detects the change in magnetic moment in an alternating magnetic field. For these embodiments, the sensor apparatus may be more sensitive to soft ferromagnetic materials.

Metal particles likely to be present in engine oil or grease will tend to be either mechanically hard ferrous materials, such as iron or steels used to form the moving parts, or mechanically soft non-ferrous materials, such as lead, tin, brass, aluminium or white metal, which may be used to hold the parts together. Ferrous materials are typically soft ferromagnetic, and for the purposes of this application the term "ferrous" will be used to describe the class of ferromagnetic materials comprising i.e. containing or consisting of iron (consistent with how it would normally be understood). Although non-ferrous ferromagnets, e.g. nickel and cobalt, also exist, most non-ferrous materials are paramagnetic or diamagnetic. Many non-ferrous materials only show a negligible paramagnetic or diamagnetic response to a magnetic field, and may essentially be classed a "non-magnetic".

The general principles behind metal detection using a magnetic field are well established, and the skilled person will understand that, and how, different metal particles may interact with an applied magnetic field. The basic concept is that an alternating magnetic field induces eddy currents in any conducting material, and will also be affected by the magnetic properties of the material. By detecting a resulting change in the magnetic field it is therefore possible to detect the presence of metals. Analysis of the result of interaction between the magnetic field and the sample may also allow classification of the metal material e.g. as ferromagnetic or non-ferromagnetic and/or a quantity of the material to be determined.

In accordance with the invention in any of its aspects or embodiments, including the further aspects which may not use printed coils, the sensing means is arranged to sense the result of interaction e.g. in a sensing region between the sample and the generated magnetic field. The sensed result may be in the form of one or more signals received by the sensing means. In some embodiments the sensing coil is arranged to generate the magnetic field for interaction with the sample, and to sense the result of the interaction between the field and the sample.

In accordance with the invention in any of its aspects or embodiments the sensing means is preferably arranged to sense a change in impedance of the conductive coil of the sensing means resulting from said interaction e.g. in a sensing region between the sample and the generated magnetic field.

In accordance with any of its aspects or embodiments, preferably the sensor apparatus includes means for generating data indicative of the sensed result of the interaction between the sample and the generated magnetic field e.g. in a sensing region. The generated data may be provided to one or more components of the sensor apparatus for use by the or each component, or to one or more components of a remote system or systems for use by thereby. The data may be transmitted to a remote system or systems for processing, storage and/or output to a user e.g. display.

As referred to herein, data indicative of the sensed result is based on the sensed result of the interaction between the sample and the generated magnetic field i.e. the one or more signals received by the sensing means. This data indicative of the sensed result may simply be indicative of the sensed result of the interaction e.g. the one or more signals received by the sensing means after interaction of the magnetic field with the sample in its raw form, or the sensed result e.g. one or more received signals may be processed to provide the output data. The method may comprise processing the sensed result of the interaction to provide the data indicative of the sensed result for output. Thus the means for generating the data may comprise one or more components e.g. one or more processors, and/or other components such as a filter etc. The generated data may be generated taking into account data obtained from one or more reference components e.g. a reference coil. Thus the generated data may be indicative of the sensed result after suitable calibration and/or balancing.

The sensor apparatus may further comprise means for transmitting the generated data indicative of the sensed result. This may enable the data to be transmitted to a remote system or systems for use thereby, or to another part of the sensor apparatus. This may be particularly useful where the sensor apparatus is located for in situ testing of a substance, where conditions may be relatively harsh. The transmission may be carried out using one or more wired or wireless connection(s), or combinations thereof. The transmission may be a wireless transmission. The method may comprise the transmitting means wirelessly transmitting the data. However, in other arrangements a wired transmission may be more appropriate. Wired transmissions may be particularly useful where the apparatus is used for in situ testing, avoiding the risk of attenuation of wireless signals or interference therewith by parts of the system e.g. pipe walls etc. This may also or additionally be useful where the apparatus is used in harsh conditions. The method may comprise the transmitting means transmitting the data using one or more wired connection(s). However it is transmitted, the data may be transmitted as or immediately once the result of the interaction between the sample and the magnetic field is sensed, or the data indicative of the sensed signal may be stored and transferred at a later time. For example, the user may download the data at a later stage for analysis.

The sensor apparatus may comprise means for providing e.g. transmitting the generated data indicative of the sensed result to one or more of; output means (e.g. a display) for outputting (e.g. displaying) information to a user based on the data indicative of the sensed result, storage means for storing data based on the data indicative of the sensed result, and processing means for using the data indicative of the sensed result in obtaining information relating to the presence of metal particles within the sample. Output means for outputting information to a user may be a display for displaying the information to a user, or an output to enable the information to be downloaded by a user etc. Preferably the output means comprises a display. The storage means, output e.g. display or processing means may operate on the received generated data indicative of the sensed result, or may carry out some processing of the data before using the data. Any processing means referred to herein may comprise a set of one or more processors.

The sensor apparatus may comprise any one or ones of the storage means, output e.g. display and processing means. Such components may be connected by wires to the sensing means. Any one or ones of these further components may form part of a sensor unit provided by the sensor apparatus. Where the sensor apparatus comprises any additional components, such as storage means, output e.g. display or processing means, such components may or may not be provided on the substrate. In some embodiments the sensor apparatus provides a sensor unit. The sensor unit may include at least the substrate, and a display. The unit may additionally comprise the aforementioned processing means and/or storage means to which the data indicative of the sensed result is provided. The unit may comprise a housing. At least some or all of the components may be located in the housing. In some embodiments the sensor apparatus may comprise one or more parts located within a fluid pipe e.g. including at least the coil of the sensing means, connected to one or more parts located on the outside of the pipe via a suitable connection e.g. via a wired connection. A storage means, output e.g. display and/or processing means may then be located outside the pipe.

In other embodiments, any one or ones of the storage means, output e.g. display and processing means may be remote from the sensor apparatus. Thus they may form part of a remote system or systems. The data may be provided to a component or components of a remote system e.g. by wireless transmission as described above. It is envisaged that the apparatus may provide the data to any one or ones of such components both forming part of the sensor apparatus and remote therefrom. The sensor apparatus may wirelessly transmit the data to the remote component(s). The remote system may be located away from a part of the system where the sensor apparatus is disposed for in situ testing e.g. away from a fluid pipe.

In any of the embodiments, and wherever it is provided, an output means may, instead or in addition to comprising a display, comprise means for making data available to a user in any way e.g. for download.

The sensor apparatus may be arranged to output the generated data e.g. to a user or to a remote system.

The method may comprise a user viewing the data output by a display (whether remote or otherwise).

Whether it forms part of the sensor apparatus or not, a storage means may comprise any suitable means for storing data indicative of the sensed result of the interaction e.g. a memory for storing the data. In this way, the data may be stored e.g. for subsequent use in determination of information relating to the presence of metal particles within the sample.

The sensor apparatus of the present invention provides sensed data i.e. sensed result of interaction between the sample and the generated magnetic field, which may be, and preferably is used in obtaining information relating to the presence of metal particles within a sample. The method may comprise using the sensed data in this way, and the method may extend to the use of the apparatus to obtain such data. The sensed result may be used together with other data in obtaining the information e.g. calibration data and/or reference data. For example, data obtained by a reference coil i.e. a reference sensing coil may be used as described below.

In accordance with the invention in any of its aspects or embodiments, including the further aspect which may not necessarily use a printed coil, the sensed data may be used to obtain the information relating to the presence of metal particles by the sensor apparatus, or by a remote system or systems using the sensed data. The information may be information relating to the presence or absence of such particles. Where the sample is a static or extracted sample the obtained information may comprise information relating to the total content of ferromagnetic e.g. ferrous metal particles within a given volume e.g. a unit volume. The total content may be a total mass. The given volume is preferably a unit volume, although could be a sample volume or sensed volume. Data by reference to a unit volume is of greatest use to a user, and is preferably obtained, and optionally provided to a user. Where the sample is a dynamic sample the information may be indicative of the sensing of individual metal particles e.g. a count thereof, and may alternatively, or more preferably additionally comprise information relating to one or more properties of at least some metal particles present in the sample. The individual metal particles sensed may be ferromagnetic particles e.g. ferrous particles, or may be non-ferromagnetic metal particles. The metal particles are sensed one at a time e.g. the passage of each individual particle through the sensing region is detected. It will be appreciated that the sensor apparatus may be configured to sense the result of interaction between the generated magnetic field and the sample to provide a sensed result for use in determining information relating to the presence of metal particle(s) of any of the types described herein.

The sensed result may be used alone, or with additional data, such as reference data, or calibration data in obtaining the information. Calibration data for use in obtaining the information may be stored e.g. by the sensor apparatus, or a remote system, and is then accessible to the processing means.

In some embodiments the sensor apparatus in accordance with any of the aspects or the invention, including the further aspects which do not necessarily use printed coils, comprises processing means for using the sensed result to obtain information relating to the presence of metal particles within the sample. The obtained information relating to the presence of metal particles within the sample may comprise information indicative of the total content of ferromagnetic e.g. ferrous particles per given volume where the sample is a static or extracted sample, or may comprise information indicative of sensing of individual metal particles e.g. a count thereof, and/or one or more properties of at least some of the sensed metal particles, where the sample is a dynamic sample.

In other embodiments the sensor apparatus in accordance with any of the aspects of the invention, including the further aspects that do not necessarily use printed coils, is arranged to generate data indicative of the sensed result and transmit the data to a remote system for use by a processing means of the system in obtaining information relating to the presence of metal particles within the sample. In accordance with the method of the present invention, the sensor apparatus may therefore be arranged to generate data indicative of the sensed result. The apparatus may be arranged to transmit e.g. wirelessly or using wires the data e.g. to remote processing means. The method may comprise receiving the transmitted data indicative of the sensed result e.g. at processing means of a remote system, and using the data to obtain information relating to the presence of metal particles within the sample.

A processing means, whether of the sensor apparatus or a remote system, may operate on the sensed result directly, or data indicative of the result generated based thereon e.g. with the raw sensed data having undergone some processing. A processing means, whether of the sensor apparatus or a remote system, may use the data indicative of the sensed result that is preferably generated by the sensor apparatus.

The processing means may be arranged to correlate the result sensed by the sensing means with: (i) the presence, (ii) the quantity and/or (iii) one or more properties of a metal particle or particles within the sample substance. The method may comprise such a step. The quantity may be a total content of ferromagnetic e.g. ferrous metal particles per unit volume where the sample is a static or extracted sample, or a count of particles where the sample is a dynamic sample.

In accordance with a further aspect of the invention there is provided a system for obtaining information relating to the presence of metal particles within a sample comprising the sensor apparatus of the invention in accordance with any of its aspects or embodiments (including the further aspects that do not necessarily use printed coils), and one or more of; remote output means e.g. a display for outputting e.g. displaying information to a user based on the data indicative of the sensed result, remote storage means for storing data based on the data indicative of the sensed result, and remote processing means for using the data indicative of the sensed result in obtaining information relating to the presence of metal particles within the sample. Where the sensor apparatus is located for in situ testing of a sample, it may be desirable for the apparatus to transmit data to a remote system e.g. computer that stores and/or processes the data. The data may be processed and/or stored over an extended period of time. Data may be transmitted to a remote system for storage and/or processing continually, or at intervals. In some embodiments the sensor apparatus may transmit data to allow real time monitoring of the content of the substance to be tested. In other embodiments, the sensor apparatus may be arranged to process the sensed data to obtain information relating to the presence of metal particles in the sample, and then provide data indicative of the obtained information to a remote system for output to a user, storage, or further processing.

Wherever the processing means for using the sensed data for obtaining the information relating to the presence of metal particles is provided, the processing means may be arranged to determine, and the method may comprise determining information relating to the presence one or more metal particles in the sample. The invention extends to using the sensor apparatus to sense the result of interaction between a generated magnetic field and a sample (received in the aperture or, in the further aspects of the invention, more generally, the sensing region), and using the sensed result of the interaction to obtain information relating to the presence of one or more metal particles in the sample in any of the manners described herein. Where the sample is a static sample the processing means may be arranged to obtain, and the method may comprise obtaining information indicative of a total content of ferromagnetic e.g. ferrous particles for a given volume e.g. per unit volume. Where the sample is a dynamic sample the processing means may be arranged to obtain, and the method may comprise obtaining data indicative of the sensing of individual metal particles e.g. a count of metal particles, and optionally one or more properties of at least some (i.e. any one or ones or each) of the sensed individual metal particle or particles in the sample. The one or more properties may comprise either, or both, of the size of the metal particles and the type of metal particles. The size of the particle may be in terms of a particle diameter or a particle mass. Determining the type of metal particle may comprise determining whether the particle is ferromagnetic, or non- ferromagnetic. Sensing individual metal particles may simply involve providing an output indicative that a particle has been detected. The sensed individual particles are sensed one at a time. However in preferred embodiments the method may comprise, and the processing means may be arranged to count the metal particles. The processing means may be arranged to provide a count of individual particles, and the method may comprise using the sensed data to determine such a count. The processing means may be arranged to determine, and the method may comprise using the data to obtain a count as a function of time e.g. per hour and/or to obtain data indicative of a change in count with respect to time. In preferred embodiments, as discussed in more detail below, the processing means is arranged to determine, and the method may comprise using the data to obtain, a count with respect to volume of fluid i.e. per given volume, such as per unit volume.

In a first preferred application the result of interaction between the sample and the generated magnetic field sensed by the sensor apparatus may be, and preferably is, used to obtain information relating to a total content per unit volume of ferromagnetic e.g. ferrous particles in an extracted sample e.g. a sample fluid. The processing means is preferably arranged to obtain such information. This may be achieved in any manner using the sensed data e.g. using data indicative of the sensed result. In these embodiments the sample may advantageously be an extracted sample held in a sample container.

It will be appreciated that the raw sensed data may be indicative of the number of ferromagnetic e.g. ferrous particles in a sensed volume defined by the sensing region. This may then be used to obtain a number of such particles per unit volume. This may be achieved using techniques known in the art, e.g. using calibration curves obtained using reference samples having a known number of the relevant type of particles. The calibration curve obtained using the reference samples may relate a number of particles determined per sensed volume (as defined by the sensing region) to a number of such particles per unit volume. Processing to convert a number of particles per sensed volume to a number of particles per unit volume may be carried out by a processing means for obtaining the data relating to the presence of metal particles using the sensed data, or may be carried out as a preliminary step of processing the sensed data e.g. to obtain data indicative of the sensed result. Data for output to a user is typically in terms of number of particles per unit volume, although it is envisaged that a number of particles per sample volume might be obtained if desired e.g. if the sample volume is known. This might be a predetermined sample volume, or a sample volume input by a user. The apparatus or a system comprising the apparatus for obtaining information relating to the presence of metal particle(s) in relation to an extracted sample may be provided pre-calibrated. A user may be required to indicate the type of material being tested in order that the correct calibration is used. The apparatus may be supplied with one or more reference samples in respective sample containers for checking the calibration. The reference samples may be of particular types of material.

In a second preferred application, where the sample is a dynamic sample i.e. defined by a volume of fluid flowing though the aperture (or, in the further aspects, more generally through a sensing region), the sensed result of interaction between the sample and the generated magnetic field sensed by the sensor apparatus may be, and preferably is used to sense, and optionally count, individual metal particles passing through the aperture (or sensing region), and optionally to further obtain information relating to one or more properties of at least some (e.g. any one or ones or each) of the particles. The processing means is preferably arranged to obtain such information. Sensing individual metal particles may simply involve providing an output indicative that a particle has been detected. The sensed individual particles are sensed one at a time. However in preferred embodiments the method may comprise, and the processing means may be arranged to, count the metal particles. The processing means may be arranged to provide a count of individual particles, and the method may comprise using the sensed data to determine such a count. The processing means may be arranged to determine, and the method may comprise using the data to obtain a count as a function of time e.g. per hour and/or to obtain data indicative of a change in count with respect to time. A change in count may indicate a worsening of a wear condition, for example. A total number of particles in the sample i.e. in the volume of fluid passing through the aperture or sensing region between first and second times of a test period may be determined. In preferred embodiments as discussed in more detail below, the processing means is arranged to determine, and the method may comprise using the data to obtain a count as a function of volume of fluid i.e. per given volume e.g. per unit volume. The processing means may be arranged to generate data indicative of sensed particles, or a count of particles, in accordance with any of the embodiments described.

Where data indicative of one or more properties of at least some of the sensed particle(s) is determined, the one or more properties may include a size and/or magnetic property or properties of the particles. The size may be a particle diameter or mass. The method may comprise using the sensed result of the interaction to categorise at least some or all of the detected individual metal particles as ferromagnetic or non-ferromagnetic. The method may comprise determining a size of at least some of the particles. The method may optionally comprise categorising the individual particles into bins e.g. depending upon particle size e.g. diameter. Each particle passing though the aperture may give rise to a signal which is sensed by the sensing means. Each such particle passes through a sensing region of the apparatus. The relatively planar coils described herein may provide a short sampling volume which is desirable when the sensor is used to determine the presence of individual metal particles passing through the aperture or sensing region. By contrast, the sampling volume for a solenoidal coil is relatively long compared to the typical dimensions of the metal particles being detected, and so most of the coil is too far away from the particle to contribute much to the measurement. There is also a greater risk that two particles will be present in the sampling volume at a given time, incorrectly being classified as a single particle, particularly for higher particle concentrations. A shorter sampling volume may increase the reliability of particle counting, as it is more likely that a signal detected will relate to a single particle, even where concentrations of particles are relatively high. A small sampling volume may also provide a higher signal to noise ratio as there will be fewer particles within the sampling volume constituting background noise.

The method extends to using the result of the interaction in any of the above manners.

The sensor apparatus may be configured to provide a sensed result of interaction between a sample and a generated magnetic field which may be used in any of the above manners. For example the sensed result may be usable to count individual metal particles in a dynamic sample provided by a volume of fluid flowing through the aperture or sensing region between first and second times, preferably with respect to volume, and optionally to further obtain information relating to one or more properties of the particles. Alternatively the sensed result may be usable to determine a quantity of ferromagnetic e.g. ferrous particles in an extracted sample, optionally wherein the quantity is a total content of the particles per unit volume.

Embodiments of the present invention have been described with respect to determining a total quantity of ferromagnetic e.g. ferrous particles, e.g. per given/unit volume of an extracted sample. The total quantity/content may be in terms of a mass. Any ferromagnetic particle present in the sensing region may contribute to the sensed interaction. The ferromagnetic particles may comprise e.g. include or consist of ferrous particles. For example, ferrous particles are most likely to be included in a sample obtained for the purposes of assessing wear. In some embodiments the total quantity is one of ferrous particles. It is envisaged that alternatively a total sensed quantity of metal particles in general (i.e. not limited to ferromagnetic particles) might be obtained e.g. per given/unit volume of an extracted sample.

Any total quantity or content of ferromagnetic e.g. ferrous particles per given e.g. unit volume referred to herein may be a total mass of the ferromagnetic e.g. ferrous particles per given e.g. unit volume.

In other embodiments relating to a dynamic sample, individual metal particles sensed may be or comprise ferrous or ferromagnetic particles. As stated above, the particles may be categorised as ferrous or non-ferrous. This may be achieved using suitable analysis of the signal(s) resulting from interaction between the sample and the magnetic field. One suitable technique is described below. Data indicative of obtained information relating to the presence of metal particles is preferably output to a user. For example the information may be displayed to a user. The method extends to such a step. This may be via a display of the sensor apparatus in some embodiments, or a remote display e.g. of a remote processing system that determined the data or otherwise.

In accordance with the invention in any of its aspects or embodiments, including those further aspects which may not necessarily use printed coils, where the sensor apparatus comprises processing means for obtaining the information relating to the presence of metal particles, the apparatus may further comprise means for transmitting data indicative of the obtained data. This may enable the data to be transmitted to a remote system or systems for use thereby. This may be particularly useful where the sensor apparatus is located for in situ testing of a substance, where conditions may be relatively harsh. The transmission may be carried out using one or more wired or wireless connection(s), or combinations thereof. The transmission may be a wireless transmission. The method may comprise the transmitting means wirelessly transmitting the data. The data may be transmitted as or immediately once obtained, or the obtained data may be stored and transferred at a later time. For example, the user may download the data at a later stage for analysis.

In accordance with any of the aspects of the invention the sensor apparatus may comprise means for providing e.g. transmitting the obtained data indicative to one or more of; output means (e.g. a display) for outputting (e.g. displaying) information to a user based on the obtained data, and storage means for storing data based on the obtained data. Alternatively or additionally it is envisaged that the data may be transmitted to processing means for further processing the obtained data. Output means for outputting information to a user may be a display for displaying the information to a user, or an output to enable the information to be downloaded by a user etc. Preferably the output means comprises a display.

The sensor apparatus may comprise any one or ones of the storage means, output e.g. display and processing means. Any one or ones of these components may form part of a sensor unit provided by the sensor apparatus. Where the sensor apparatus comprises any additional components, such as storage means, output e.g. display or processing means, such components may or may not be provided on the substrate. In some embodiments the sensor apparatus provides a sensor unit. The sensor unit may include at least the substrate, and a display. The unit may additionally comprise the aforementioned processing means and/or storage means to which the data indicative of the sensed result is provided. The unit may comprise a housing.

In other embodiments, any one or ones of the storage means, output e.g. display and processing means may be remote from the sensor apparatus. Thus they may form part of a remote system or systems. The data may be provided to a component or components of a remote system by wireless transmission as described above. It is envisaged that the apparatus may provide the data to any one or ones of such components both forming part of the sensor apparatus and remote therefrom. The sensor apparatus may wirelessly transmit the data to the remote component(s). The invention extends to a system comprising the sensor apparatus having processing means for obtaining the information relating to the presence of metal particles, and means for transmitting data indicative of the obtained data, and means remote from the sensor apparatus for outputting e.g. displaying data indicative of information relating to the presence of metal particles in a sample received from the apparatus and/or storing such data.

The sensor apparatus of the apparatus in any of its aspects or embodiments may further comprise any one or ones of various additional components.

The sensor apparatus may comprise one or more reference component for calibrating or balancing the sensing means. The method may comprise using an output of the or each reference component in obtaining the information indicative of the presence of metal particles using the sensed result of interaction. One or more said reference component may comprise a reference coil printed on the substrate, preferably wherein the reference coil is identical to the printed coil of the sensing means (in those aspects or embodiments using a sensing means having a printed coil). Because the coils are printed onto a substrate, it is relatively simple to manufacture identical coils. This is not the case with mechanically wound coils. A reference coil may be located adjacent the sensing coil. A reference coil may be printed on the same substrate as the conductive coil that constitutes the sensing means, but spaced apart from it such that it does not circumferentially surround the aperture that receives the sample substance. In this way the reference coil can be made as similar as possible to the sensing coil so that it may effectively balance the sensing coil. By identical, it is meant that the coil has the same number and arrangement of coil portion(s) as the sensing coil, with each coil portion being of the same construction as the corresponding coil portion of the sensing coil. Preferably the reference coil circumferentially surrounds a further different aperture formed in the substrate i.e. different to the aperture surrounded by the coil for receiving the test sample. The further aperture is preferably of the identical to i.e. of the same size and shape as the aperture surrounded by the coil of the sensing means. Where multiple such reference coils are provided, each surrounds a different respective aperture. The, each or a reference coil may sense the result of interaction between a magnetic field and a respective reference sample of a substance e.g. fluid containing no metal particles. The reference sample may be located within the aperture around which a reference coil extends in the same manner as the sample to be tested, e.g. using a sample container e.g. test tube. The method may comprise locating such a reference sample in the aperture. In dynamic sample testing, a reference sample of fluid may be provided in the aperture associated with the reference coil by arranging for fluid to pass through the aperture after passing through a filter arranged to remove metal particles. Alternatively, the, each or a reference component e.g. coil may be arranged to provide a background measurement without reference to a sample substance e.g. fluid. The reference component e.g. coil may then effectively sense the result of interaction between a magnetic field and the surrounding material(s) e.g. air and/or the substrate material. The reference component may be arranged so as not to contact or interact with the substance e.g. fluid to be tested. It will be appreciated that one or more reference components may be provided, which may be of the same or different types. The one or more reference component may alternatively comprise an electrical component of equivalent value to the sensing coil.

The sensor apparatus may include multiple reference components e.g. reference coils. For instance, the sensor apparatus may contain one, two, three or more reference components e.g. reference coils or other components.

The reference component(s) and the coil of the sensing means may be connected to a common current supply. The reference component(s) and the coil of the sensing means may together constitute a standard bridge or half-bridge circuit. The output of the bridge circuit may be, or may be proportional to, the difference in impedance between the reference component and the coil of the sensing means. Using a balanced bridge circuit may help to reduce or remove any signal fluctuations not associated with the sample substance e.g. fluid, such as electrical noise from the drive circuit, temperature variations, etc. The output of the bridge, i.e. the impedance imbalance, may be amplified and converted with an analogue-to-digital converter for storage, further processing or display to a user.

Where the sensing coil forms part of a bridge circuit, as described above, information relating to the presence of metal particles that is obtained using the sensed result of the interaction, and which is optionally displayed to a user, may be indicative of a difference in bridge output with and without a sample present. The value may be multiplied by an appropriate scaling factor. For instance, the scaling factor may convert the measured bridge output into the total content ferromagnetic e.g. ferrous material per unit volume, e.g. based on pre-determined calibration data.

In accordance with the invention in any of its aspects described herein including a substrate, the sensor apparatus may further comprise a thermally insulating material for insulating the substrate. The substrate may be coated with and/or surrounded by the thermally insulating material. The thermally insulating material may be e.g. a foam material, such as a polymer or glass-based foam. In these ways the sensor can be made less sensitive to temperature fluctuations. This may be particularly important in certain applications where the sensor may be exposed to relatively high temperatures, e.g. where the sensor is used to monitor engine oil. Thermal expansion of the substrate may cause the position of the coil(s) to shift in use, which could reduce the sensitivity or resolution of the sensor apparatus, and is therefore desirably avoided. However, depending upon the materials used for the substrate and/or the intended application, thermal insulation may not be necessary.

Desirably, only the magnetic field of the coil of the sensing means couples with the sample substance. However, there will also be some electric fields associated with the coil. Accordingly, in some embodiments electrostatic shielding is disposed between the sensing coil and the sample to reduce potential electric field coupling between the coil and the sensing region. Such shielding may additionally be disposed between any reference coil and a sample associated therewith. This may help to reduce sensitivity to variations in the dielectric constant of the substance being tested. In some embodiments the sensor apparatus may further comprise electrostatic shielding disposed between the conductive coil of the sensing means and the aperture, preferably wherein the electrostatic shielding comprises an incomplete ring printed on the substrate. The incomplete ring may be printed between the innermost coil turn and the aperture. This electrostatic shielding should be connected to ground, and may be arranged to prevent or reduce current flow. For instance, the electrostatic shielding may be arranged so as to not form a complete loop or may be formed from a material with high resistivity. Where the electrostatic shielding is printed as an incomplete ring on the substrate it may advantageously be printed at the same time using the same process as the or each conductive coil of the substrate. Electrostatic shielding may additionally be disposed between any reference coil and a sample associated therewith. This may be of any of the forms described in relation to the sensing coil.

The sensor apparatus in accordance with any of the embodiments described herein may contain external shielding for reducing interference from external magnetic fields or radiation. This shielding may surround at least the sensing means e.g. the sensing coil, and preferably at least the sensing means and any reference components of the sensor apparatus. The shielding may comprise a conductive shield, box or grid. For instance, the sensor apparatus may comprise an aluminium shield surrounding the sensing coil.

In contrast to US 6,051,970, it is desirable for the sensor apparatus in accordance with any of the embodiments described herein to contain no ferrite shielding. It has been recognised that the presence of ferrite shielding does not necessarily provide any significant improvement in performance. In fact, it has been found that a ferrite shield may provide a mechanism whereby temperature changes or mechanical disturbances can couple to the magnetic field sensed by the coil and hence introduce additional interference or noise that would not otherwise be present. The non-linear magnetic behaviour of ferrite may cause coupling between magnetic fields at different frequencies so that the sensor is affected by DC and low frequency magnetic fields e.g. from motors, power lines etc., to which it would otherwise be immune. The ferrite shielding may also add to the bulk and cost of the sensor apparatus. Preferably therefore the sensor apparatus contains no ferrite shielding. Where the apparatus forms part of a sensor unit, the sensor unit may contain no ferrite shielding.

The sensor apparatus in accordance with any of the aspects or embodiments of the invention may further comprise a housing. The substrate is then mounted with respect to the housing. The substrate may be mounted at least partially, and optionally entirely within the housing. The housing preferably comprises an opening to enable a sample to be received by the aperture of the sensor apparatus. The opening should be configured so that the apparatus may be used in whichever of the above described manners is intended. For example, the opening may be configured to receive a sample container, or to receive or connect to a fluid pipe of a system for mounting the apparatus thereto such that the fluid flows through the aperture as discussed above. In other embodiments, the opening may be in the form of a passage through which a dynamic fluid sample may flow to pass through the aperture (or, in other embodiments, more generally a sensing region) and, where provided, to contact a flow sensor. The opening may or may not therefore extend completely through the unit. The opening may be a slot or the housing may be moulded to define such an opening.

Where the sensor apparatus forms part of a sensor unit, the unit e.g. the housing thereof preferably comprises an opening aligned with e.g. coaxial with the aperture of the substrate (or the sensing region). This may enable a sample container to be located in the aperture of the sensor apparatus or to enable the unit to be mounted to a fluid pipe, or, more generally when testing a dynamic fluid sample, to enable the fluid to pass through the aperture or sensing region, and preferably to contact a flow sensor. In some embodiments the apparatus e.g. unit comprises a fluid conduit extending through the aperture and defining first and second ends to the exterior of the housing for connection to a section or sections of a fluid pipe.

In accordance with the invention in any of its aspects or embodiments, the sensor apparatus may be in the form of a sensor unit. In these embodiments the apparatus preferably comprises a housing. The unit may be a portable unit. This may enable it to be carried to a site, and a sample then extracted and tested on site. The sample may also be extracted and then tested off-site. In other embodiments the unit may be located with respect to e.g. mounted to a system for in situ testing as described above.

In some embodiments the unit is a portable unit, and the sensor apparatus of the unit comprises processing means for using the sensed result of the interaction to obtain information relating to the presence of metal particles within the sample (which may be carried out in accordance with any of the embodiments earlier described), and a display for displaying information based on the obtained information to a user. This may be particularly suitable where the sample is a static or extracted sample, in which case the information obtained, and displayed to a user, is information relating to a total content per given (e.g. unit or sensed) volume of ferromagnetic e.g. ferrous particles in the sample. The unit may then provide a self-contained system for obtaining information relating to the presence of metal particles. The unit preferably further comprises an opening for receiving a sample container comprising a sample to be tested e.g. a test tube.

In accordance with a further aspect of the invention there is provided;
a sensor unit comprising;
a housing;
and a sensor apparatus for use in obtaining information relating to the presence of metal particles within a sample of a substance to be tested, the sensor apparatus comprising:
a PCB substrate comprising an aperture for receiving a sample of a substance to be tested, the substrate having one or more electrically conductive coils printed thereon; magnetic field generating means for generating a magnetic field for application to a sample received in the aperture; and
sensing means for sensing the result of interaction between the generated magnetic field and a sample received in the aperture, the sensed result of the interaction being usable to determine information relating to the presence of metal particle(s) in the sample;
wherein the substrate includes an electrically conductive coil printed thereon that circumferentially surrounds said aperture for receiving the sample, the electrically conductive coil forming part of said sensing means;
the sensor unit further comprising an opening coaxial with the aperture of the substrate for receiving a sample container comprising a sample to be tested, processing means for using the sensed result of the interaction to obtain information relating to the presence of metal particles within the sample, and a display for displaying information based on the obtained information to a user. Preferably the information obtained, and displayed to a user, is information relating to a quantity of ferromagnetic e.g. ferrous particles in an extracted sample, preferably a total content per given e.g. unit volume.

The present invention in any of the further aspects may include any or all of the features described in relation to any of the other aspects of the invention to the extent they are not mutually inconsistent. In other embodiments the sensor unit may not comprise means for obtaining the information relating to the presence of metal particles, but may be configured to transmit the data to remote processing means for obtaining the information using the sensed result of the interaction.

In accordance with a further aspect of the invention there is provided a sensor unit comprising;
a housing;
and a sensor apparatus for use in obtaining information relating to the presence of metal particles within a sample of a substance to be tested, the sensor apparatus comprising:
a PCB substrate comprising an aperture for receiving a sample of a substance to be tested, the substrate having one or more electrically conductive coils printed thereon;
magnetic field generating means for generating a magnetic field for application to a sample received in the aperture; and
sensing means for sensing the result of interaction between the generated magnetic field and a sample received in the aperture, the sensed result of the interaction being usable to determine information relating to the presence of metal particle(s) in the sample;
wherein the substrate includes an electrically conductive coil printed thereon that circumferentially surrounds said aperture for receiving the sample, the electrically conductive coil forming part of said sensing means;
the sensor unit further comprising an opening coaxial with the aperture of the substrate to enable the unit to be mounted with respect to a system using a fluid to be tested in a manner such that the fluid flows through the aperture of the substrate, means for generating data indicative of the sensed result of the interaction, and either;
processing means for use in obtaining information relating to the presence of metal particles within a sample of a substance to be tested, or means for transmitting the data to remote processing means for use in obtaining information relating to the presence of metal particles within a sample of a substance to be tested.

The data may be transmitted wirelessly. The sensor unit is therefore used for in situ i.e. online testing of a fluid. Preferably the sensed data is indicative of a count of individual metal particles in the sample defined by the volume of fluid flowing through the aperture in a test period. The apparatus may comprise a fluid conduit extending through the aperture and having ends for connection to a section or sections of a fluid pipe as described earlier, or may be arranged so as to be mounted to a pipe of a system with the pipe extending through the aperture. The present invention in any of the further aspects may include any or all of the features described in relation to any of the other aspects of the invention to the extent they are not mutually inconsistent. In particular the sensor apparatus may be in accordance with any of the embodiments described herein, and may form part of a sensor system or unit as described herein.

Of course, units for use with extracted samples may not include processing means to determine the information relating to metal particles in the sample, and may transmit data indicative of the sensed result to remote processing means. Similarly units for in situ testing may include processing means, rather than transmitting data to remote processing means to obtain the information.

The sensor apparatus or system comprising the same of the invention in any of its aspects or embodiments may also comprise any additional suitable circuitry for manipulating or processing the data output by the sensing means indicative of the interaction between the sample and the magnetic field and/or for obtaining desired information relating to the presence of metal particles with a sample based on the output data. For instance, the apparatus system may comprise circuitry for amplifying or filtering the signal. The apparatus system may comprise phase sensitive detection circuitry for tuning out any off-frequency noise. Such additional circuitry may or may not be provided on the substrate of the sensor apparatus, or within a sensor unit defined thereby.

It may be advantageous to place any further electrical circuitry on a separate substrate laterally spaced away from the sample. This is because the sample may be relatively hot. However, where a reference component is used this should generally be located on the same substrate as the sensing coil so that it experiences essentially the same conditions. Alternatively, it may be desirable to include all of the circuitry constituting the sensor apparatus or system onto a single substrate, for instance, for size or manufacturing reasons. Thus, at least the sensing means is provided on the substrate. Any separate components of the magnetic field generating means, and any other components may be provided on the same substrate or another substrate, which may be of the same or different type as the first. Of course, further components need not be provided on a substrate.

Also disclosed, by way of example, is a method of manufacturing a sensor apparatus in accordance with any of the embodiments described. The method may comprise;
providing a substrate comprising an aperture; and
printing one or more coils onto the substrate, such that at least one of the coils circumferentially surrounds the aperture.

The method may comprise a step of forming the aperture in the substrate. Where the substrate is a multilayer substrate the method may comprise forming an aperture in each layer of the multi-layered substrate. This step may occur at any time before or after a coil is printed onto the substrate. The or each coil may comprise one or more coil portions located on respective levels of the substrate. The method may comprise printing the or each coil portion in a spiral pattern. The coil may be a single layer coil or a multilayer coil comprising coil portions at different levels in the substrate. The method may further comprise printing a second, substantially identical, coil onto the substrate for use as a reference and/or printing an incomplete ring between the coil and the aperture for use as an electrostatic shield.

The method may comprise providing a first layer of substrate comprising an aperture;
printing one or more first coil turns onto the first layer to provide a coil portion, such that the one or more first coil turns circumferentially surround the aperture;
providing a second layer of substrate comprising an aperture;
printing one or more second coil turns onto the second layer to provide a second coil portion, such that the one or more second coil turns circumferentially surround the aperture;
joining the first and second layers together to form a multi-layered substrate comprising an aperture; and
electrically connecting the first and second coil portions, for instance with a via. The aperture of the multi-layered substrate is provided by the apertures of the first and second layers. The layers are joined with the apertures coaxial.

Alternatively or additionally a multilayer coil may be provided by printing sets of one or more coil turns defining respective coil portions on to different sides of a given substrate around an aperture therein.

The method of manufacture described in relation to this example may include steps of manufacturing or manufacturing connections to any or all of the features described in relation to any of the other aspects of the invention to the extent they are not mutually inconsistent. In particular the method of manufacture may be used to manufacture a sensor apparatus in accordance with any of the embodiments described herein.

As described above, the sensor apparatus or systems of the present invention may advantageously be used to count individual metal particles in a sample fluid, wherein the sample is a dynamic sample provided by a volume of fluid flowing through the aperture between first and second times (i.e. the second preferred application). These embodiments are used for in situ testing i.e. online testing of a fluid. For this application, it has been found that it may be desirable to determine a count of individual metal particles passing through the aperture with respect to volume of fluid e.g. per given volume of fluid. For example, the count may be expressed in terms of number of particles counted per volume of fluid tested i.e. the volume of the sample passing through the aperture between first and second times, or more preferably per unit volume of fluid, such as per ml, or per 100ml. In contrast to determining a count with respect to time e.g. per unit time, or between the first and second times, or over any other period, it has been found that a count with respect to volume may be more useful in at least some contexts. Determining a count with respect to volume may allow results to be obtained from online testing that are more easily comparable to results obtained in a laboratory, which are typically per unit volume. It will be appreciated that the volume of fluid flowing through the aperture in a test period between first and second times, e.g. per day, will typically be significantly greater than the unit volumes that are typically used in laboratory and other measurements. A count with respect to volume e.g. per unit volume enables easier comparison with other counting methods, and to ensure that reliable and consistent results are obtained under different operating conditions, it is often important to know what volume has been examined. Thus, the method according to these embodiments may further comprise determining the count of individual metal particles as a count per given volume of fluid e.g. per unit volume. The method may comprise generating and optionally outputting data indicative of such a count. That is, any references herein to a count, may, in embodiments, refer to such a count with respect to volume. In order to obtain such a count, it is necessary to determine directly or indirectly a volume of sample fluid flowing through the aperture (or, in further aspects which may not use a printed coil surrounding an aperture, more generally the sensing region) between the first and second times i.e. over the test period to which the count relates. This will correspond to the volume of the sample tested. The count of individual metal particles with respect to volume of fluid is based on the count of individual metal particles detected in a given time period e.g. between first and second times defining the dynamic sample passing through the aperture or sensing region, and the volume of fluid in the sample. The count of individual metal particles is a cumulative total over the time period.

While in some cases it might be assumed that the rate of flow of fluid through the aperture (or more generally sensing region) is equivalent to the rate of flow of process fluid through a part of the system in which the sensor apparatus is disposed for testing, this assumption may not be appropriate, in particular in preferred embodiments in which the sensor apparatus samples only a fraction of the total fluid flowing through the part of the system e.g. pipe. The flow rate of fluid may, in any case, vary widely across the part of the system e.g. pipe. It is desirable, therefore, to determine more accurately the rate at which fluid may be considered to be flowing through the aperture (or sensing region). Preferably the method comprises using a flow sensor to determine data indicative of a rate of flow of fluid flowing through the aperture (or sensing region). The sensor apparatus may comprise the flow sensor, or may form part of a system including the sensor.

It is believed that incorporating a flow sensor into a sensor apparatus to enable counting of individual metal particles within a dynamic flow as a function of volume passing through a sensing region of the sensor is novel and advantageous in its own right, independent of the type of sensor apparatus or system being used to sense the individual metal particles. In other words, the sensor apparatus or system need not include the printed sensing coil surrounding an aperture of the earlier aspects of the invention.

Thus, in accordance with a further aspect of the invention there is provided a sensor apparatus for use in determining a count of individual metal particles with respect to volume for a dynamic sample provided by a volume of fluid flowing through a sensing region of the apparatus between first and second times, the apparatus comprising:
magnetic field generating means for generating a magnetic field for application to a sample in the sensing region;
sensing means comprising one or more electrically conductive coils for sensing the result of interaction between the generated magnetic field and a dynamic sample provided by a volume of fluid flowing through the sensing region between first and second times;
and a flow sensor for determining data indicative of the rate of fluid flow through the sensing region;
the sensed result of the interaction and the data indicative of the rate of fluid flow through the sensing region determined by the flow sensor being usable in determining a count of individual metal particles with respect to volume for the dynamic sample.

The present invention in this further aspect may include any or all of the features described in relation to any of the other aspects of the invention to the extent they are not mutually inconsistent. The present invention in this further aspect may be combined with and is compatible with any of the features of the sensor apparatus or systems described above in relation to any of the other aspects, and to any of the methods described above to the extent that they are not mutually incompatible.

As mentioned earlier, the first and second times may or may not be predefined, and may be any two times e.g. a past time and current time during continual monitoring.

In this further aspect of the invention, the one or more electrically conductive coils of the sensing means may be of any suitable construction. In preferred embodiments a single coil is provided, although in other embodiments multiple coils may be used. In contrast to the earlier aspects of the invention, the or each coil of the sensing means may be a wound coil, such as a solenoid. The wound coil is preferably a helically wound coil. The or each coil of the sensing means may be a non-printed coil. Any suitable construction may be used for the or each coil. Desirably, the magnetic field at the centre of the sensing region is substantially parallel to an axial direction of the coil. In embodiments, the wound coil is not a toroidal coil.

However, preferably, as in the earlier aspects of the invention, the sensing means comprises a printed coil. In this further aspect of the invention, the sensing means is preferably of the construction of the sensing means in accordance with any of the earlier aspects or embodiments of the invention. The sensing means, and, indeed the sensor apparatus, may include any of the features described in relation to the earlier aspects of the invention. Preferably the apparatus comprises a substrate comprising an aperture for receiving a sample of a substance to be tested, the substrate having one or more electrically conductive coils printed thereon, and the magnetic field generating means is arranged for generating a magnetic field in use for application to a sample received in the aperture; and the sensing means is arranged for sensing the result of interaction between the generated magnetic field and a sample received in the aperture in use, the substrate including an electrically conductive coil printed thereon that circumferentially surrounds the aperture for receiving the sample, the electrically conductive coil forming part of said sensing means. The sensing means may include a single printed electrically conductive coil. The sensing means, coil, aperture and substrate etc. may be in accordance with any of the embodiments of the earlier aspects of the invention. The or each portion of the printed coil is therefore preferably a spiral coil portion. The or each coil portion is preferably not a toroidal coil portion. Desirably, the magnetic field at the centre of the aperture is substantially parallel to a thickness dimension of the substrate. This is the axial direction with respect to the aperture or coil. The magnetic field at the centre of the aperture may be substantially perpendicular to the plane of the first and second surfaces of the substrate. As mentioned above, the use of a printed coil to provide the sensing means is advantageous in the context of determining a count of individual metal particles in a dynamic sample, as the coil may then define a relatively short path in the direction that fluid flows through the aperture i.e. a relatively short sensing region.

The sensing region is the region in which the sensing means is arranged to sense the result of interaction between the generated magnetic field and the sample in the sensing region. The extent of the region will be defined by the or each coil of the sensing means. Where the or each coil of the sensing means is a wound coil, the sensing region defined may comprise a region defined by the interior space of the or each coil. Where sensing means uses a printed coil surrounding an aperture in a substrate on which the coil is printed, the sensing region may comprise the space defined by the aperture as described in the earlier embodiments. For other types of sensing coil, a sensing region may similarly be defined.

In these further aspects of the invention, the magnetic field generating means preferably comprises one or more electrically conductive coil. The coil(s) of the magnetic field generating means may or may not be the same coil(s) that provide the sensing means. The magnetic field generating means may further comprise means for providing a supply of current to the or each coil of the magnetic field generating means. The magnetic field generating means may comprise a single coil. As with the earlier aspects of the invention, desirably the same coil or coils are used to generate the applied magnetic field and to sense the result of interaction between the sample and the generated magnetic field. In some embodiments a single coil is provided which is the only coil of the sensing means and the magnetic field generating means e.g. a single printed coil. Nonetheless, it is envisaged that separate coils may be used to generate the applied magnetic field and to sense metal particles. It is also contemplated that an applied magnetic field may be generated in some other way, not necessarily utilising a coil. Where the sensing means comprises a printed sensing coil as in the earlier aspects and embodiments of the invention, the magnetic field generating means may be of the construction of any of the embodiments described in relation to those embodiments.

The present invention also extends to a method of using the apparatus in accordance with the further aspect of the invention in any of its embodiments in determining information for use in obtaining a count of individual metal particles with respect to volume for a dynamic sample provided by a volume of fluid flowing through a sensing region of the apparatus between first and second times. The method may comprise: using the sensing means to sense the result of interaction between the dynamic sample and an applied magnetic field, using the flow sensor to determine data indicative of the rate of fluid flow through the sensing region, wherein the sensed result of the interaction and the flow rate data are usable to determine a count of individual metal particles with respect to volume for the dynamic sample e.g. per unit volume. The method may further comprise obtaining the count of individual metal particles with respect to volume for the dynamic sample e.g. per unit volume. The method then comprises using the sensed result of the interaction and the flow rate data to determine the count of individual metal particles with respect to volume for the dynamic sample. The invention therefore extends to using the apparatus in accordance with the further aspect of the invention in any of its embodiments in obtaining a count of individual metal particles with respect to volume for a dynamic sample provided by a volume of fluid flowing through a sensing region of the apparatus between first and second times. The method may comprise: using the sensing means to sense the result of interaction between the dynamic sample and an applied magnetic field, using the flow sensor to determine data indicative of the rate of fluid flowing through the sensing region, and using the sensed result of the interaction and the flow rate data to determine a count of individual metal particles with respect to volume for the dynamic sample. As discussed above, the step of using the sensed result and flow rate data to determine the count of metal particles with respect to volume may be carried out by processing means remote from the sensor apparatus. The method may comprise disposing the sensor apparatus such that a fluid to be tested flows through the sensing region of the apparatus, and using the magnetic field generating means to generate a magnetic field for application to the sample. As described earlier, the method in any of the aspects or embodiments in which a count of individual metal particles is determined may comprise determining information relating to one or more properties of at least some of the metal particles.

The present invention in these further aspects may include any or all of the features described in relation to any of the other aspects of the invention to the extent they are not mutually inconsistent. The present invention in this further aspect may be combined with and is compatible with any of the features of the sensor apparatus or systems described above in relation to any of the other aspects, and to any of the methods described above to the extent that they are not mutually incompatible.

Any reference herein to determining a count of metal particles with respect to volume is preferably a count of metal particles with respect to unit volume.

The method of using the sensor apparatus may comprise the sensor apparatus transmitting data indicative of the sensed result of the interaction and the data indicative of the flow rate to remote processing means which uses the data in determining the count of metal particles with respect to volume e.g. per unit volume.

In these further aspects, the sensor apparatus may comprise means for generating data indicative of the sensed result of the interaction between the sample and the generated magnetic field, and means for generating data indicative of the flow rate sensed by the flow sensor. The means may be a set of one or more processors. The sensor apparatus may comprise means for providing the generated data indicative of the sensed result and the flow rate data to one or more of; output means e.g. a display for outputting information to a user based on the data indicative of the sensed result and/or the flow rate data, storage means for storing data based on the data indicative of the sensed result and/or data based on the flow rate data, and processing means for using the data indicative of the sensed result and the flow rate data in obtaining information indicative of a count of metal particles in the sample with respect to volume. The sensor apparatus may comprise any one or ones of said storage means, output or processing means. However, in preferred embodiments any one or ones of the storage means, output or processing means may be remote from the sensor apparatus, wherein the sensor apparatus further comprises means for transmitting the generated data indicative of the sensed result and the flow rate data thereto. It will be appreciated that the apparatus in accordance with the further aspects of the invention may be arranged to generate, transmit, output, or use data indicative of the sensed result and the flow rate data in the same manner described in respect of the earlier aspects or embodiments in relation to the data indicative of the sensed result.

The sensor apparatus of these further aspects may comprise processing means for using the sensed result of said interaction and the data indicative of the flow rate in obtaining data indicative of the count of individual particles in the sample with respect to volume e.g. per unit volume. However, it given that the sensor apparatus is for in situ testing, it may be more appropriate for the sensor apparatus to transmit the sensed data to a remote processing means for use in obtaining such data. In preferred embodiments the sensor apparatus is arranged to generate data indicative of the sensed result of the interaction and data indicative of the flow rate, and to transmit the generated data to remote processing means for use in determining a count of individual metal particles in the sample with respect to volume. The data may be transmitted in any suitable manner e.g. using a wired connection or wirelessly. Preferably the data is transmitted using a wired connection. A set of one or more wires may be connected to the sensor apparatus for this purpose. The present invention extends to a system comprising the sensor apparatus of this aspect of the invention in any of its embodiments, and processing means remote from the sensor apparatus for using the sensed result of said interaction and the flow rate data to determine a count of individual metal particles in the sample with respect to volume.

The apparatus in accordance with these further aspects may comprise a reference component for calibrating or balancing the sensing means. The reference component is preferably located adjacent the sensing means. The reference component may be of the construction described in respect of the earlier aspects of the invention. In some preferred embodiments in which the sensing means comprises a coil printed on a substrate and surrounding an aperture, the reference component comprises a reference coil printed on the substrate and surrounding an aperture, which is preferably identical to the printed coil of the sensing means. However, as discussed above, the reference components need not surround an aperture in which a sample is provided, and may not be arranged to sense interaction with a fluid sample. The apparatus may further comprise external shielding or thermal insulation as described in respect of the earlier aspects of the invention.

In accordance with any of the further aspects or embodiments of the invention including a flow sensor, regardless of the construction of the sensing means i.e. whether or not a printed coil is used, it will be appreciated that the sensed result of the interaction between the generated magnetic field and the sample, and the data determined by the flow sensor, may be, and preferably are, used to determine a count of individual metal particles in the sample with respect to volume e.g. per unit volume. The determined count of individual metal particles will be based upon the count i.e. the cumulative total of individual metal particles sensed in the test period. The test period is the period may be the period between first and second times defining the dynamic fluid sample that flows through the sensing region or aperture. This processing may be carried out by the sensor apparatus, or the sensor apparatus may provide data indicative of the sensed result and the flow rate determined by the flow sensor to external processing means for determining the count with respect to volume. The method of the present invention in its various aspects or embodiments may comprise using the sensed result of the interaction between the generated magnetic field and the sample, and the data determined by the flow sensor to determine a count of individual metal particles in the sample with respect to volume e.g. per unit volume. As described in relation to the further aspects of the invention, for any of the aspects or embodiments of the invention relating to a sensor apparatus including a flow sensor, and which apparatus is for use in determining a count of individual metal particles with respect to volume, the apparatus is preferably arranged to generate data indicative of the sensed result of the interaction and data indicative of the flow rate, and transmit the data to remote processing means (preferably using a wired connection) for determining the count of individual metal particles. The method may involve the apparatus performing such steps, and determining the count using data received from the apparatus at remote processing means.

It will be appreciated that in the discussion of those aspects and embodiments of the invention in which a count of individual metal particles with respect to volume is determined for a dynamic sample, the dynamic sample is, in preferred embodiments in which the sensing means comprises a coil surrounding an aperture, defined by the volume of fluid passing through the aperture between first and second times. More generally, where the apparatus does not use sensing means of this construction, the dynamic sample is defined by the volume of fluid passing through the sensing region between first and second times. Certain preferred features of these aspects of the invention relating to determining the count with respect to volume may therefore refer to the sensing region or aperture, with the aperture being applicable in those preferred embodiments in which such an aperture is present, and surrounded by a printed coil.

In accordance with any of the aspects or embodiments of the invention including a flow sensor, (including the earlier aspects using a printed sensing coil, and the later aspects which may not necessarily use such a coil), the flow rate measured by the flow sensor may be taken to correspond to the rate of flow of fluid through the aperture or sensing region, and hence may be used to determine at least an approximation of the volume of fluid passing through the aperture or sensing region between the first and second times i.e. the sample volume. The flow sensor should be positioned suitably such that it may be assumed to experience the same flow rate as that through the aperture or sensing region. The flow sensor may be provided adjacent the aperture or sensing region e.g. upstream or downstream thereof. The flow sensor is preferably located out of the aperture or sensing region. The method may then comprise using the determined rate of flow and the data indicative of the sensed result of interaction between the generated magnetic field and the sample in the sensing region or aperture to determine a count of metal particles with respect to volume e.g. per given or unit volume. In embodiments in which the sensor apparatus comprises a sensing coil printed on a portion of a substrate and surrounding an aperture, the flow sensor may be provided on the substrate. However, this need not be the case. It will be appreciated that there are various ways in which the flow rate may be used to estimate the volume of fluid passing through the aperture or sensing region between first and second times. For example, the flow rate may be used with the cross-sectional area of the aperture or sensing region to determine a volume per unit time. The total volume passing through the aperture or sensing region between the first and second times may be determined by consideration of the flow rate with respect to time over the test period between the first and second times. In preferred embodiments the sensor apparatus forms part of a sensor unit, the sensor unit further comprising the flow sensor.

In any of the aspects or embodiments using a flow sensor, the flow sensor may be of any type, such as a thermal or pressure sensor. In some preferred embodiments the sensor is a thermal sensor. A flow sensor as used herein refers to a sensor for determining a rate of flow of fluid.

Of course, in other contexts, it may still be desirable to instead determine a count of individual metal particles as a function of time e.g. per given or unit time, such as per unit time e.g. per day, hour, minute etc. In such embodiments, it is not necessary to provide a flow sensor.

In any of the aspects or embodiments of the invention in which the sensor apparatus is used to determine a count of individual metal particles in a dynamic sample, whether or not with respect to volume, as discussed above, the sensor apparatus is mounted in use such that the fluid to be tested flows through the aperture, or, in the further aspects of the invention which do not necessarily use such an aperture, the sensing region. A portion of the sensor apparatus including the aperture or sensing region, may be immersed in the fluid so as to contact the fluid. For example, in embodiments in which a substrate defines an aperture which is surrounded by a sensing coil, a portion of the substrate defining the aperture may be immersed in fluid so as to contact the fluid. The one or more coils of the sensing means, whatever their construction, may or may not be immersed in fluid so as to contact the fluid. Where provided, a flow sensor is of course also immersed in the fluid so as to contact the fluid.

The sensor apparatus in accordance with any of the aspects or embodiments of the invention for determining a count of individual metal particles, is preferably mounted with respect to a system in which a fluid to be tested is used in a manner such that the fluid flows through the aperture or sensing region to provide a dynamic sample. The method may comprise mounting the sensor apparatus in such a manner. Preferably the aperture or sensing region is configured to receive only a portion i.e. a fraction of the fluid flow of the system in the region of the sensor apparatus. The sensor apparatus is preferably configured to sample only a portion of the fluid flow through the part of the system to which the apparatus is mounted. The method may comprise mounting the apparatus in such a manner. Preferably the sensor apparatus is mounted with respect to a fluid pipe of a system so that fluid flowing along the pipe flows through the aperture or sensing region to provide the sample, and preferably the sensor apparatus is then mounted such that only a portion i.e. a fraction of the fluid flowing through the pipe passes through the aperture or sensing region. Thus, in these embodiments, the aperture or sensing region samples only a portion of the fluid flowing through the pipe. This may be achieved by selecting the size of the sensor apparatus and particularly the size of the aperture or sensing region of the sensing coil to be relatively small compared to the diameter of the part of the system e.g. pipe. As mentioned above, it has been found that by providing a relatively small sensor that is arranged to sample only a fraction of a total flow in the region of the sensor, the sensor apparatus may be able to detect smaller metal particles that are in suspension in the fluid, and which may give an early indication of a wear problem. While the present invention may be used to sense the entire flow of fluid through a part of a system e.g. a pipe, and may use any suitable diameter sensing coil, the use of the printed sensing coil in accordance with certain aspects, and preferred embodiments of the further aspects of the invention, enables the dimensions of the coil of the sensing means to be minimised, making it particularly suitable for sensing only a portion of a fluid flow through a system. It will be appreciated that the duration of a test period i.e. the period between first and second times, may be selected as appropriate taking into account the size of the sensing region or aperture, and flow speeds experienced, to provide a suitable sample enabling a count to be obtained with a desired degree of confidence.

Of course, in some embodiments, the sensor apparatus may be mounted such that the aperture or sensing region receives the entire fluid flow of the system in the region of the sensor apparatus. For example, the sensor apparatus may be mounted such that the aperture or sensing region surrounds a fluid pipe through which fluid flows. The fluid pipe may or may not be a bypass or other sampling pipe. The sensor apparatus may be mounted in any suitable manner with respect to a fluid pipe so that fluid flowing along the pipe flows through the sensing region or aperture to provide the sample. The fluid thus flows through the sensing region. The sensor apparatus may be mounted around the pipe, or may be mounted between sections of the pipe and/or at an end of the pipe. The sensor apparatus may be mounted such that the pipe is received by the sensing region or aperture. Some possible embodiments are discussed above.

The following construction of a sensor unit comprising the sensor apparatus is applicable to any of the aspects or embodiments of the invention in which the sensor apparatus is for use in obtaining a count of metal particles, whether with respect to volume or time, and whether or not a flow sensor is provided. The features are applicable whether or not the sensing means is of the preferred construction comprising a coil printed on a substrate. The sensor unit may enable the sensor apparatus to be effectively mounted with respect to a system such that a dynamic fluid sample is provided in the aperture or sensing region.

The sensor apparatus is configured such that fluid flows through the sensing region or aperture in use to provide the fluid sample. In use a portion of the sensor apparatus is disposed so that fluid flows through the sensing region or aperture. The method may comprise mounting the sensor apparatus with respect to a system using the fluid to be tested so that fluid flows through the sensing region or aperture. A portion of the sensor apparatus, and, in some embodiments, only a portion of the apparatus, may be immersed in the fluid flow so as to contact fluid to enable fluid to flow through the sensing region or aperture. The portion may include the portion of a substrate of the sensor apparatus comprising the aperture where the apparatus comprises a substrate having an aperture surrounded by a sensing coil, or more generally, the portion of the apparatus comprising the sensing region. The portion that is immersed so as to contact fluid may optionally include the sensing coil or coils. The portion of the sensor apparatus that is immersed preferably includes a flow sensor in the preferred embodiments in which the sensor apparatus includes a flow sensor so that the flow sensor is immersed in the fluid flow so as to contact the fluid. In some embodiments a portion of a substrate of the sensor apparatus (i.e. which defines an aperture surrounded by a sensing coil) may include the flow sensor. A (further) portion of the sensor apparatus may be disposed out of contact with the fluid. It will be appreciated that, provided fluid may pass through the aperture or sensing region, the or each coil, or indeed substrate, where provided, need not contact the fluid. For example, the coil and aperture may surround a tube through which fluid may flow to pass through the sensing region as described below. The further portion may alternatively or additionally include any processors, connecting wires etc.

In preferred embodiments the sensor apparatus forms part of a sensor unit, the sensor unit having a first portion intended to be immersed in a fluid to be tested in use, and configured such that when the first portion is immersed in fluid, fluid may pass through the aperture or sensing region of the sensor apparatus to provide a dynamic sample. Thus the first portion of the sensor unit is located in the path of fluid flow. The first portion of the sensor unit may comprise the portion of the sensor apparatus having the or each coil of the sensing means, and may be arranged to house this portion of the sensor apparatus. The first portion of the sensor unit may or may not be configured such that the coil(s) of the sensing means contact the fluid in which the first portion is immersed. The first portion may seal certain portions of the apparatus from contact with fluid. In some embodiments only the first portion of the sensor unit is immersed in fluid in use.

In those aspects of the invention and the preferred embodiments in which the sensing means comprises a coil printed on a substrate, the first portion may include (and may house) the portion of the substrate comprising the aperture with the coil of the sensing means therearound. The first portion is configured such that fluid may flow through the aperture. The first portion may be configured such that the portion of the substrate surrounding the aperture is immersed in fluid so as to contact the fluid, and optionally such that the sensing coil surrounding the aperture is immersed in fluid so as to contact the fluid.

The first portion preferably includes (and may house) a flow sensor in the preferred embodiments in which a flow sensor is provided, and is configured such that when the first portion is immersed in the fluid to be tested, the flow sensor contacts the fluid. The flow sensor is preferably located adjacent the sensing coil(s). The flow sensor may be located upstream or downstream of the sensing coil(s). The flow sensor may be located out of the sensing region or aperture. In some embodiments the flow sensor may be provided on a portion of a substrate having the sensing coil(s).

The first portion of the sensor unit may be provided by an exposed relevant portion of the sensor apparatus e.g. the sensing coil(s). However, in preferred embodiments the first portion of the sensor unit comprises a housing. The first portion of the sensor unit then houses at least a relevant portion of the sensor apparatus that is to be immersed in fluid so as to contact the fluid. The housing is configured such that when the first portion is immersed in a fluid to be tested in use, fluid contacts the relevant portion of the sensor apparatus. The housing is configured such that when the first portion is immersed in a fluid to be tested in use, fluid passes through the aperture or sensing region (and where applicable, contacts the flow sensor) as described above.

The first portion of the sensor unit may be in the form of an elongate probe that is inserted into the fluid flow in use. The first portion may be cylindrical, although any suitable shape may be used. The sensor unit may define a proximal end and a distal end, with the first portion being located at the distal end.

In particularly preferred embodiments (regardless of the construction or content of the first portion), the first portion of the sensor unit comprises a passage therethrough, through which fluid may flow in use in order to pass through the aperture or sensing region of the sensor apparatus to provide the dynamic sample, and, where applicable to contact the flow sensor. Preferably the first portion of the sensor unit comprises a housing, and the passage is a passage through the housing. The passage may be concentric with the or each sensing coil, and/or the aperture of the sensor apparatus where provided. The passage may be a bore extending through a housing of the first portion of the sensor unit from one side of the first portion to an opposite side thereof. Regardless of the construction of the first portion of the sensor unit, the first portion of the sensor unit may comprise a single such passage. Any portion of the sensor apparatus intended to be immersed in fluid in use so as to contact the fluid may then be located within the passage for contacting fluid. In some embodiments the or each coil of the sensing means may be disposed in the passage for contacting fluid in use. Alternatively, the or each coil may be located around the passage. In these embodiments the or each sensing coil does not contact the fluid. In this case the passage may be defined at least in part by a tube extending through the sensing region or aperture of the or each coil of the sensing means. The tube may extend through the first portion. The tube may or may not be integral with a remainder of the first portion e.g. of a housing defining the first portion. In preferred embodiments in which the sensing means comprises a coil printed on a substrate and surrounding an aperture, the tube may pass through the aperture. In such embodiments, the substrate does not contact the fluid. In other embodiments in which the sensor apparatus comprises a substrate comprising an aperture surrounded by a sensing coil, the passage may be defined at least in part by a portion of the substrate defining the walls of the aperture. The portion of the substrate may be a portion extending between first and second sides thereof. In these embodiments, the aperture defines at least a part of the passage. The sensing coil will then be located around the passage as in the embodiments in which a tube extends through the sensing region.

However the passage is provided, where a flow sensor is provided, the flow sensor is preferably located in the passage. This ensures that the flow sensor can be assumed to experience the same flow rate as that through the aperture or sensing region i.e. that through the passage. The passage preferably has a cross sectional area that is smaller than a cross sectional area of a part of or for a system through which the fluid to be tested flows, and in respect to which it is mounted in use e.g. of a pipe. This may ensure that only a portion of the fluid flow in the system e.g. pipe, enters the passage, and hence that only a portion of the fluid flow is sampled by the sensor apparatus (and, where appropriate, flow sensor).

The entire sensor unit may be intended to be immersed in the fluid in use. In preferred embodiments the sensor unit preferably has a second portion that is intended to be disposed out of contact with the fluid to be tested in use. In such embodiments, the second portion may house components not intended to contact fluid flow and seal them from contact with the fluid. The second portion may house other components of the sensor apparatus e.g. any reference components, processors etc. The second portion may house any reference component e.g. one or more reference coils of the sensor apparatus. This may enable the reference components to experience corresponding conditions to the sensing means e.g. temperature etc., but without contacting or even sampling the fluid. Of course, the reference component(s) may alternatively be located in the first portion of the sensor unit. They may then be arranged to be disposed so that they do not contact or even sample fluid. However, in some embodiments in which the reference component(s) include one or more reference coils surrounding an aperture, the sensor unit is arranged such that fluid flows through the aperture, with filter means being provided in order to remove metal particles from the fluid that passes through the aperture in order to provide a reference sample. It will be appreciated that reference components may be disposed in any suitable manner, provided that they experience the same environment as the sensing coil(s). A similar arrangement may be provided where the sensing coil does not surround an aperture, e.g. is in the form of a wound coil. The or each reference component may be an identical coil. A filter may be provided to remove metal particles from fluid passing through the sensing region of the or each reference coil. In some embodiments a reference coil is located adjacent the or each coil of the sensing means. In some embodiments only the first portion of the sensor unit is intended to be immersed in fluid in use.

The sensor unit may be mounted or mountable with the first portion located to the interior of a part of or for a system comprising a fluid to be tested for immersing the first portion in the fluid in use, and with the second portion located to the exterior of the part of the system. The part of the system may be any suitable part through which fluid will flow in use e.g. a pipe, fluid conduit or container of a system. In some preferred embodiments the part is a pipe. The method may comprise mounting the sensor unit in such a manner.

In some embodiments the method comprises mounting the sensor unit with the first portion located to the interior of a part of a system comprising a fluid to be tested for immersing the first portion in the fluid in use, and with the second portion located to the exterior of the part of the system. In other embodiments the sensor unit may be provided mounted to a part for a system comprising a fluid to be tested, and the method may then comprise connecting the part to the system so that a fluid to be tested is present in the part of the system in use. For example, in some embodiments the sensor unit is provided mounted to a section of pipe for the system. The part e.g. pipe section may then be mounted to a system comprising the fluid to be tested. The sensor unit may be permanently or releaseably mounted to the part. For example, the sensor unit may be welded thereto. The part may comprise one or more connectors for connecting the part to a system. For example, where the part is a section of pipe, the pipe may comprise connectors at one or both ends thereof. The or each connector may comprise a flange or a threaded portion, for example. The method may comprise mounting the part to such a system.

Preferably the first portion of the sensor unit is disposed or arranged to be disposed through an opening in a wall of the part of or for the system comprising a fluid to be tested to immerse it in the fluid in use and with the second portion remaining outside the wall. The method may then comprise disposing the first portion through an opening in a wall in this manner. In other embodiments in which the sensor unit is provided mounted to a part for a system, the first portion may be provided disposed through an opening in a wall of the part in this manner. The first and second portions of the sensor unit may therefore be configured to be disposed on the interior and exterior sides of a wall of a part of or for a system comprising a fluid to be tested in use. The second portion of the unit may have a greater diameter than the first.

In some preferred embodiments the sensor unit comprises means for retaining the unit mounted with respect to an opening in the wall of the part of or for the system with the first portion disposed through the opening in a wall and with the second portion remaining outside the wall. The retaining means is preferably configured to prevent the second portion from passing through an opening in the wall of a part of or for the system while permitting the first portion to pass therethrough. The retaining means may be arranged to engage the periphery of the opening in the wall on the exterior thereof, or to engage an edge of a mounting component associated with the part e.g. inserted through an opening in the wall, for retaining the unit mounted in this manner. The retaining means may comprise a shoulder or flange or any similar formation. More than one retaining means may be provided e.g. multiple shoulders. This may enable the retaining means to cooperate with different diameters of opening, and/or additionally with mounting components associated with the part e.g. a mounting collar associated with an opening in the part. The retaining means is preferably located between the first and second portions of the unit. For example, a shoulder may be defined at the end of the second portion closest to the first portion. While the retaining means is preferably an integral part of the sensor unit, separate retaining means may be used. It will be appreciated that other forms of retaining means may alternatively or additionally be used e.g. one or more fasteners, adhesives, welding etc.

Whether or not it is provided ready mounted to the part, the sensor unit may be arranged to provide a fluid tight seal with the opening of the part when disposed therethrough to prevent the passage of fluid around the edges thereof between the interior of the part and the exterior thereof through the opening in use. The method may comprise mounting the sensor unit so as to provide such a seal.

The sensor unit may alternatively or additionally comprise mounting means to facilitate mounting of the unit to a part of a system. The mounting means may be located between the first and second portions of the unit. Where mounting means is provided, a retaining means e.g. shoulder as described above may be defined by the mounting means. Retaining means e.g. a retaining shoulder, may be provided at one or both ends of the mounting means. For example, in some embodiments, a first retaining shoulder is defined between the first portion and the mounting means, and a second retaining shoulder is defined between the mounting means and the second portion of the unit.

The mounting means may be a mounting ring or collar. In some embodiments the mounting means may have a diameter that is greater than that of the first portion of the unit and less than that of the second portion of the unit. Retaining shoulders may be defined at each end thereof between the first portion and the retaining means and the second portion and the retaining means respectively. The mounting means may be configured to be received by a portion of the part. For example, the mounting means may be received by an opening in the wall of a part of the system to which the sensor unit is mounted, or by a mounting component associated with the part. Such a mounting component may be e.g. a mounting collar associated with the part, such as a mounting collar welded to the part. The mounting means of the sensor unit may engage the periphery of an opening through a wall of a part of a system to which the sensor unit is mounted between the interior and exterior surfaces of the wall, or a periphery of a mounting component associated with the part that is inserted in the opening between the inner and outer ends thereof. The inner end refers to the end of the mounting component closest to the interior of the part. The mounting means of the sensor unit may comprise a threaded portion for cooperating with the portion of the part, or may be arranged to provide an interference fit with the portion of the part. The mounting means may be configured to provide a fluid tight seal with an opening in the part to prevent the passage of fluid around the edges thereof between the interior of the part and the exterior thereof through the opening. This may be the case whether the mounting means cooperates directly with the wall of the part, or a mounting component associated with the part. Additional sealing means may be provided around the mounting means.

In any of the aspects or embodiments of the invention in which the sensor apparatus is arranged to test a dynamic sample of fluid of a system, the method may further comprise disposing mixing means in the system upstream of the sensor apparatus e.g. sensor unit to agitate the fluid flow before it reaches the sensor apparatus e.g. sensor unit. This may help to ensure that the portion of the fluid flowing through the sensor apparatus is representative of the overall fluid flow. For example, a static mixer may be used. The invention extends to a system comprising the sensor apparatus or unit and such mixing means. In some embodiments in which the sensor apparatus is provided mounted to a part for a system e.g. a pipe section, the part may comprise such mixing means. The mixing means may be of any suitable type e.g. a static mixer. The mixing means is disposed upstream of the sensing region to ensure that fluid flow is agitated before reaching the sensing region.

In accordance with a further aspect of the invention there is provided a method of determining a count of individual metal particles with respect to volume for a dynamic sample, the method comprising:
applying a magnetic field to a sample received in a sensing region defined by one or more electrically conductive coils; and
using the one or more electrically conductive coils to sense a result of interaction between the generated magnetic field and a dynamic sample provided by a volume of fluid flowing through the sensing region between first and second times;
using a flow sensor to determine data indicative of the rate of fluid flow through the sensing region;
and using the sensed result of the interaction and the data indicative of the rate of fluid flow through the aperture determined by the flow sensor being to determine a count of individual metal particles with respect to volume for the dynamic sample.

In accordance with a further aspect of the invention there is provided a sensor apparatus for use in determining a count of individual metal particles with respect to volume for a dynamic sample, the apparatus comprising:
a PCB substrate comprising an aperture for receiving a sample of a substance to be tested, the substrate having one or more electrically conductive coils printed thereon;
magnetic field generating means for generating a magnetic field in use for application to a sample received in the aperture; and
sensing means for sensing the result of interaction between the generated magnetic field and a dynamic sample provided by a volume of fluid flowing through the sensing region between first and second times;
wherein the substrate includes an electrically conductive coil printed thereon that circumferentially surrounds said aperture for receiving the sample, the electrically conductive coil forming part of said sensing means;
the apparatus further comprising a flow sensor for determining data indicative of the rate of fluid flow through the aperture;
the sensed result of the interaction and the data indicative of the rate of fluid flow through the aperture determined by the flow sensor being usable in determining a count of individual metal particles with respect to volume for the dynamic sample.

The present invention in these further aspects may include any or all of the features described in relation to any of the other aspects of the invention to the extent they are not mutually inconsistent. The present invention in accordance with the further apparatus aspect of the invention may include any of the feature described in relation to the aspects or embodiments of the invention for determining a count of individual metal particles in a dynamic sample using a flow sensor, and may be used in any of the manners described. The sensing means, substrate, one or more coils, and magnetic field generating means etc. may be in accordance with any of the earlier described aspects or embodiments.

In accordance with the invention in any of its aspects, once data indicative of a count of individual metal particles has been determined, whether with respect to time or volume, the data may be used in various manners. In preferred embodiments the data is used to draw inferences about wear of the system through which the fluid has passed. The fluid may, as described above be taken from an engine, such as a marine engine etc. When the number of particles counted e.g. per given time or volume, exceeds a given threshold, corrective action may be taken or the system may be shut down.

The methods in accordance with the present invention may be implemented at least partially using software e.g. computer programs. It will thus be seen that when viewed from further aspects the present invention provides computer software specifically adapted to carry out the methods herein described when installed on one or more data processors, a computer program element comprising computer software code portions for performing the methods herein described when the program element is run on one or more data processors, and a computer program comprising code adapted to perform all the steps of a method or of the methods herein described when the program is run on a data processing system. The one or more data processors may be a microprocessor system, a programmable FPGA (field programmable gate array), etc.

Generally, the present invention in any of the aspects described above may include any or all of the features described in relation to any of the other aspects of the invention to the extent that they are not mutually inconsistent and do not depart from the scope of the appended claims.

Various embodiments of the present invention will now be described by way of example only, and by reference to the accompanying drawings in which:
Fig. 1 schematically shows the coil arrangement in a conventional sensor apparatus;
Fig. 2 schematically shows the coil arrangement in an embodiment of the present invention;
Fig. 3 shows the coil arrangement similar to that of Fig. 2 from an alternate angle;
Fig. 4 shows a system including a coil arrangement like that shown in Figs. 2 and 3;
Fig. 5 shows one example of a product including a sensor apparatus according to the present invention;
Fig. 6 shows another example of a product including a sensor apparatus according to the present invention;
Fig. 7A is a perspective view of a sensor unit in accordance with another embodiment of the present invention;
Figure 7B is a cross sectional view of the sensor shown in Figure 7A;
Fig 8 shows the sensor unit of Figs. 7A and B mounted within a section of pipe;
Fig. 9 shows an alternative mounting of the sensor unit of Figures 7A and B; and
Fig. 10 shows a pipe section including the sensor unit of Figures 7A and B and a stator mixer.

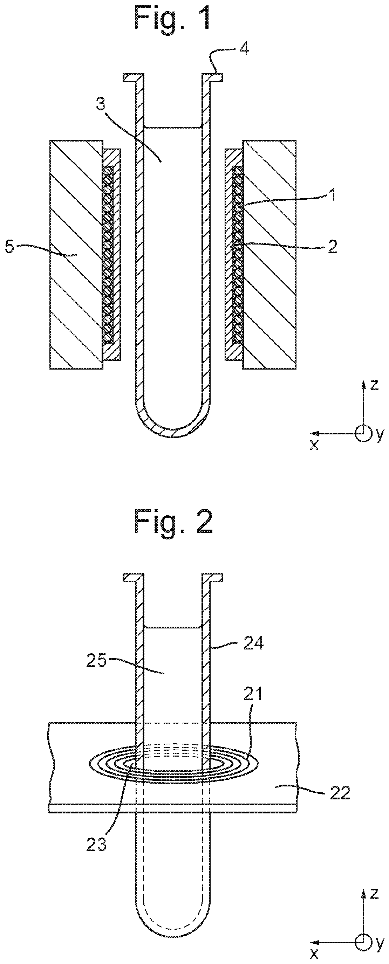

Fig. 1 illustrates a sensor apparatus like that described in US 6,051,970. A solenoidal coil 1 is driven with current to generate a magnetic field which interacts with metal particles suspended in a fluid 3 contained within a sample test tube 4. The coil 1 is surrounded by ferrite shields 5 which act to confine the lateral extent of the generated field. For clarity, further details of the housing or shielding have been omitted. A ferrite shield is formed from ferrite, which is a ceramic material composed of iron oxides.

It can be seen that the coil 1 has a relatively long axial extent, and that each turn of the coil 1 varies continuously in each of the x, y and z directions. To manufacture such a coil, wire must be mechanically wound onto a bobbin 2. The mechanical winding process is fundamentally time consuming, and the operation and parts associated with the machining are relatively expensive. The winding process may also suffer from a lack of reproducibility from coil to coil, particularly where operating conditions change. This may in particular make it difficult to manufacture a suitable reference coil, and where a wound reference coil is used it may be necessary to include additional electrical components to ensure that the coil 1 can be appropriately balanced. Of course, any deviations or mechanical instabilities of the coil could lead to a reduction in sensitivity of the apparatus if not accounted for.

Fig. 2 illustrates the coil arrangement for a sensor according to embodiments of the present invention, with a sample provided in a test tube located in the aperture thereof. Other features may be provided on the substrate which are not shown in Figure 2. Figure 2 shows the portion of the substrate in the vicinity of the sensing coil and the aperture which it surrounds. In the present invention, instead of mechanically winding a solenoidal coil of wire, as shown in Fig. 2 the coil 21 is printed directly onto a flat substrate 22. The coil 21 provides both the sensing means and magnetic field generating means. The coil 21 is printed in a circular spiral pattern surrounding a circular aperture 23 formed in the substrate 22. The substrate 22 may be a standard printed circuit board formed of glass-reinforced plastic. For instance, the substrate 22 may comprise glass fibres embedded in a plastic matrix, e.g. an epoxy resin matrix, a polyimide matrix or a PTFE matrix. However, it will be understood that other materials may suitably be used as a substrate, for instance, plastics or ceramics. The substrate 22 should ideally have a thermal coefficient of expansion suitable to provide stability under intended operating conditions. The substrate 22 should be rigid enough to support the coil and to avoid mechanical or thermal deformations in use. The printed coil 21 may be etched out of a layer of e.g. copper, or may be printed directly onto the substrate 22 using conductive ink. Other suitable printing techniques may also be used to pattern the coil 21 onto the substrate 22.

The aperture 23 is sized to receive a sample test tube 24 containing a suspension of metal particles 25. The test tube is shown in Figure 2. It will be appreciated however that a sample may also be introduced by any other suitable means. In particular, a sample may be introduced as a continuous flow through a pipe in which case the aperture 23 may be sized so that it may be mounted about the pipe, or otherwise to sample a desired portion of the flow through the pipe. The example described will be in relation to ferrous metal particles, which are most commonly found in a sample obtained from a system for the purposes of assessing wear.

As with Fig. 1, an electrical current is supplied to the coil 21 to generate a magnetic field. The generated field is strongest (and primarily directed along the z axis) at the centre of the coil 21, i.e. within the aperture 23. The field strength decreases with distance away axially from the coil 21. As the coil 21 is essentially planar, and relatively short compared to the solenoidal coil shown in Fig. 1, the sensing region of the Fig. 2 sensor apparatus within which sensing coil 21 is adequately sensitive to sense the result of interactions between ferrous particles and the generated field, is relatively small so that the suspension of metal particles 25 extends throughout and beyond the sensing region. A sensed volume is defined. This is the volume of the sample within the sensing region, and whose interaction with the magnetic field may be detected. The magnitude of the generated magnetic field, and hence the sensitivity of the sensor apparatus, will depend on the number of turns of the coil. In the present invention the number of turns can be increased by printing the coil 21 in a spiral pattern.

The number of turns can be further increased by using a multi-layered substrate with coil portions being printed onto more than one, or each layer, with the portions of the coil on adjacent layers being connected through a via. Coil portions may also be printed onto different sides of one layer. The 'coil' therefore comprises multiple coil portions on different levels, i.e. layers or sides, of the substrate. Naturally, in this case the coil turns on different levels should be such that the current flows in the same sense on each layer, such that the magnetic fields generated by each turn reinforce each other. The layers of the substrate are joined to one another e.g. laminated to provide a unitary substrate. Each coil portion may have any number of turns. The coil portion on the surface shown in Fig. 2 is a spiral coil. Preferably, each of the coil portions is a spiral coil.

In these ways, the number of coil turns can be increased if desired to a comparable number of an extended solenoid coil whilst still being confined to the substrate. When a multi-layer substrate it used, the number of layers is not particularly limited, but standard 8-layer printed circuit boards may suitably be used. Printed circuit boards are available with 16, 24, *etc.* layers. It will be understood that a standard n-layer printed circuit board includes n conductive layers, but also includes a number of non-conductive, e.g. spacer or core layers (consistent with how the term "layers" is normally used in relation to printed circuit boards). Any known multi-layer board architecture may suitably be used to form the substrate. It is envisaged that multiple standard printed circuit boards may also be laminated together to provide a thicker printed circuit board and hence substrate. Of course, it is not essential that the substrate is multi-layered, and a single layer substrate may be used. By way of example, an 8 turn spiral may be printed onto each layer of an 8-layer board to form a 64 turn coil. This may provide a good balance between cost and sensitivity. In one exemplary embodiment the printed tracks defining the turns of each coil portion are about 0.2 mm wide, and the spacing between the turns is also about 0.2 mm. For an 8 turn spiral coil this gives a total coil annular width of about 3 mm.

Where a sample is introduced through the aperture 23 in sample test tube 24 as shown in Fig. 2, it has been found that aperture diameters in the range between about 1 and 2 cm, for instance between 1.25 cm and 1.5 cm may be suitable. However, the diameter of the aperture may vary widely, particularly if the sample is introduced through a flow pipe.

The sensor apparatus of the present invention may also include shielding to reduce interference from external magnetic fields or radiation, or from metal particles external to the sample, but for reasons of clarity this is not shown in Fig. 2. The shielding may take the form of a conductive shield, grid or box surrounding the coil and sample. Because the coil is relatively small, e.g. compared to the solenoidal coil shown in Fig. 1, the shielding can be placed nearer to the coil without causing currents or fields in the shield to interfere with the measurement. Typically, the shielding may be disposed about 2 or 3 coil diameters away from the sample. The grid of box will typically be made from a non-ferrous metal, e.g. aluminium. This serves to shield the sensor from alternating fields. The shielding has no substantial effect on static fields, but these also do not effect the measurement. The measurement is also not significantly affected by alternating fields at frequencies away from the frequency at which the coils are driven as these can easily be tuned out at the signal processing stage.

It is noted that unlike US 6,051,970 the sensor illustrated in Fig. 2 does not include any form of ferrite shielding. It is in fact preferred in the present invention to not use ferrite shielding because it has been found, unexpectedly, that this may actually reduce the performance of the sensor apparatus. The advantage of ferrite shielding is that since it is not very conductive it can generally be placed closer to the sample than typical non-ferrite shielding. However, ferrite shielding is not itself very effective at shielding external fields. Furthermore, because the ferrite shielding has non-linear magnetic behaviour, static external fields, or off-frequency alternating fields, which would normally have no effect on the measurement, may interact with the ferrite material to produce spurious signals at or near to the measurement frequency. Additionally, because the ferrite shield has a high magnetic permeability, any movement of the ferrite shield e.g. due to mechanical disturbances or temperature fluctuations may in turn generate an alternating field. Thus, the ferrite shield may itself provide new mechanisms for introducing noise or interference to the measurement. Removing the ferrite shielding also allows a further reduction in bulk, and cost, of the sensor apparatus. It is also noted that standard sizes and shapes of ferrite shielding are not readily compatible with the substrate-based coil arrangements described herein and incorporation of such shielding would require modification of the standard shielding adding to processing complexity.

An electrostatic shield may also be disposed between the coil and the sample to reduce electric field coupling between the coil 21 and the sample, and hence to reduce sensitivity to variations in the dielectric constant of the fluid being tested. The electrostatic shield is connected to ground and arranged to avoid or reduce any current flow that would otherwise act to cancel the applied magnetic field. For example, the shield may be arranged so as to not form a complete loop. Alternatively, the shield may form a complete loop provided it is made of a high resistance material. The shield may be provided as a separate sheet of material to the coil/substrate or may be formed from an extra copper track on each PCB layer. For instance, the shield may take the form on an incomplete ring on each PCB layer within the spiral coil that is connected to ground. This may be the most practical arrangement and can be readily integrated with the manufacturing process for the coil. Such a shield may not be required when the test fluid shows little variation in dielectric properties, e.g. oils, but is more useful where there could be a mixture of different fluids e.g. oil containing droplets of water (water has a very high dielectric constant of around 80, whereas the dielectric constant for a typical oil is only around 2.4). The electrostatic shield may be most useful when the sensor apparatus is used to count individual particles in a continuous flow of sample fluid, where the sample fluid may be more likely to contain a (potentially varying) mixture of fluids.

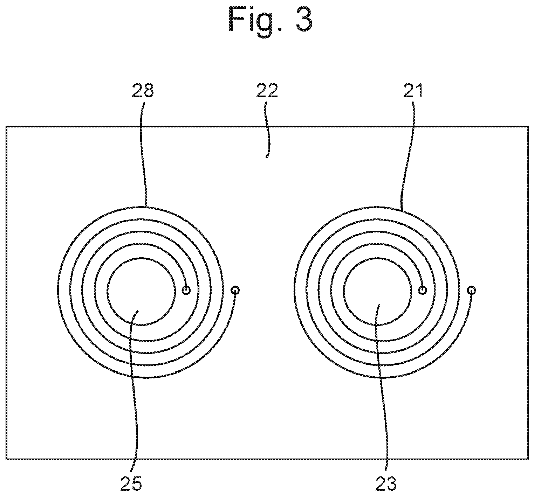

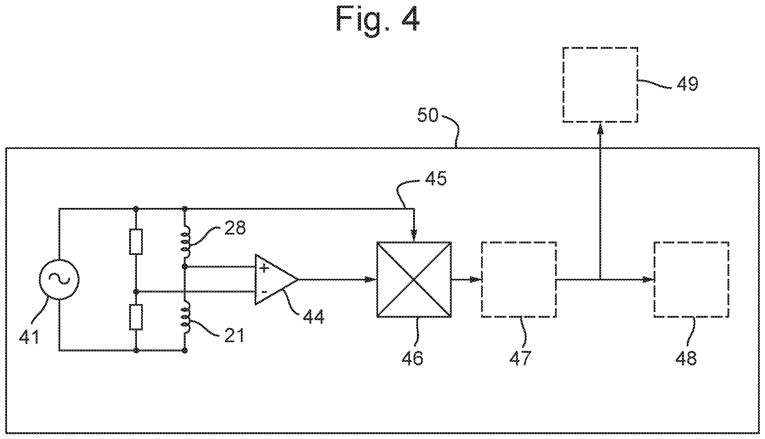

Fig. 3 shows a coil arrangement similar to that described in relation to Fig. 2 from a top-down perspective. In this embodiment, the substrate 22 is printed with two spaced-apart coils: a sensing coil 21 as described above, and a reference coil 28 that is used to calibrate the measurements. Optionally, the reference coil 28 circumferentially surrounds a second aperture 25. The reference coil 28 may be arranged to monitor a reference sample, or may simply be used as an electrical dummy. Preferred measurement techniques are similar to those set out in US 6,051,970 and described in relation to Fig. 4 below, where the coils 21 and 28 together form one half of a bridge circuit, and are both driven by a single A.C. current supply. The bridge may be driven by a digitally synthesised sinusoid. The other half of the bridge circuit is formed by two resistors, and the bridge may further comprise a digital potentiometer for adjusting the balance. In this arrangement, the output of the bridge circuit represents the difference in impedance between the sensing coil 21 and the reference coil 28. The use of printing facilitates the manufacture of a reference coil 28 that is identical to the sensing coil 21, which in turn facilitates balancing of the bridge such that the output is zero when no sample is present. Any change in conditions affecting both coils equally will not lead to an output. It will be appreciated that multiple reference components or coils may be used and that the invention is not limited to the particular type of bridge circuit described herein. For instance, the substrate may include one sensing coil 21 and three reference coils. It will also be appreciated that the reference coil 28 need not be used, for instance an electrical component having the same electrical characteristics as the sensing coil 21 could be used instead. Furthermore, the sensor apparatus need not have any reference components.

Fig. 4 illustrates some of the components of an exemplary system employing the sensor apparatuses described herein. A common source 41 provides an alternating signal to the sensing coil 21 and reference coil 28, which together form a half-bridge circuit as described above. This circuit senses the result of an interaction between the sample fluid and the sensing coil 21. The signal detected by this circuit may be passed through a number of signal processing stages. For example, as shown in Fig. 4, the detected signal is first passed through a differential amplifier 44. The amplified signal and a phase reference signal 45 are then passed to a phase sensitive detector 46 to help tune out any off-frequency noise.

The processed signal output from the phase sensitive detector 46 is indicative of the signal detected by the sensing circuit comprising the sensing coil 21 and the reference coil 28. The processed signal may then optionally be provided to any of a processing unit 47, a display 48, an internal storage unit (not shown), or to an external computer 49. For instance, the processed signal may first be passed through an internal processing unit 47 before being provided to a display 48 within the housing. Alternatively, or additionally, the processed signal may be passed to an external computer 49 for processing and/or display in which case either or both of the internal processor 47 and the display 48 may be omitted. In this case the unit may also comprise transmitting means for providing the signal to the external computer 49. The signal may be provided through a wired or wireless connection. The processed signal may also be provided to an internal storage means, for instance, so that it may be accessed or downloaded after a measurement is complete. For example, data indicative of the signal detected by the sensing coil 21, or data indicative of the processed signal output from the phase sensitive detector 46, may be passed to a removable memory for subsequent transfer to an external computer. Alternatively, the data may be stored on an internal memory that is accessible via a USB port, or other suitable connection (including wireless). Any or all of these components (other than the external computer 49) may be contained within the same housing 50 as the sensor apparatus, as shown in Fig. 4, or may be provided externally. Any external components may be situated remote from the housing, and may be connected wirelessly or through a network, or may be situated local to the housing and e.g. connected by a cable.

It will be appreciated that the arrangement of components illustrated in Fig. 4 is not intended to be limiting and any suitable circuitry may be used for measuring a change in the sensing coil 21 responsive to a sample being introduced or for providing an output to the user. It will also be appreciated that although the details of some components are not shown (for brevity), the circuitry may also include any suitable filtering, amplifying or demodulating circuitry, for example, any of the circuitry described in US 6, 051,970 (e.g. in Fig. 1 thereof).

When a sample containing metal particles is introduced through the aperture 23 into the sampling volume of the sensor, i.e. into the magnetic field generated by the sensing coil 21, the metal particles will magnetically couple to the coil 21. This interaction will result in a measureable change in the coil impedance. There are a number of factors that may cause the coil impedance to change and these are known in the art.

A first contribution results from the alternating magnetic field inducing eddy currents in the conductive metal particles, which in turn produces a secondary magnetic field which may be detected by the sensing coil 21. Since all metals are conductive, these effects will always occur although for some materials they may be relatively weak and contribute little to the measurement. The contribution resulting from this effect will generally increase with particle size.

The imaginary, or reactive, part of the coil impedance will also be influenced by changes in inductance of the coil due to the magnetic properties of the sample. For instance, ferromagnetic e.g. ferrous materials will act to reinforce the generated field and increase the magnetic induction experienced by the sensing coil 21. This will cause an increase in the inductance of the sensing coil 21, and hence its impedance, relative to the reference coil 28.

For non-ferromagnetic magnetic materials the magnetic coupling may be weaker and a combination of different effects may contribute to the measured impedance.

For the bridge circuit described in relation to Fig. 4 above, a change in inductance of the sensing coil 21 relative to the reference coil 28 produces an alternating signal at the junction of the coils, with the amplitude and phase of this signal being measured relative to the reference signal 45 at the phase sensitive detector 46. The contribution resulting from the increase in magnetic induction due to the presence of ferromagnetic e.g. ferrous particles will generally be at the same frequency and in phase with the reference signal (with the coils connected as shown in Fig. 4). Changes in impedance resulting from eddy current induction will generally result in a signal at the same frequency as but out of phase with the reference signal 45, for instance, for small particles and/or low measurement frequencies this signal may be 90 degrees out of phase with the reference signal 45, and for progressively larger particles, or higher measurement frequencies, the phase will change until it is eventually 180 degrees out of phase with the reference signal 45, i.e. acting to oppose the ferromagnetic effect and reduce the inductance of the sensing coil 21.

In general the total signal generated by the sensor apparatus for subsequent storage, processing or transmission results from the sum of both of the effects described above. The amplitude and phase of this signal will depend on the sensed interaction between the magnetic field and the metal particles, i.e. on the size, the conductivity and the magnetic permeability of the particles within the sample. The generated signal may thus be analysed by decomposing the signal into two sinusoidal signals each at the measurement frequency, one signal being in phase with the reference signal 45 and the other being 90 degrees out of phase. The in-phase component includes the ferromagnetic contribution, and for sufficiently small particles the in-phase component essentially only includes the ferromagnetic contribution. In this case, the change in inductance, and hence the amplitude of the signal, increases linearly with the amount of ferromagnetic e.g. ferrous material. The out-of-phase component includes the contribution resulting from the induced eddy currents. The relative amplitudes of the two components will vary depending on the nature of the sample, i.e. on which effects are contributing to the sensed change in impedance.

Two main applications that make use of these techniques will now be described.

The first application involves measuring the total content of ferrous particles per unit volume of sample of fluid, typically provided in ppm (parts per million) or mg/ml. For this application, the sample fluid is preferably introduced in a sample test tube. As discussed above, the introduction of ferrous particles will cause an increase in coil impedance. It can generally be assumed that the magnetic coupling between the ferromagnetic moments and the applied field will dominate the measurement so that other, relatively weak, contributions can be ignored. Provided that the sample extends throughout the entire sensing region (and preferably extends axially beyond the sensing region) the sensed interaction will result from the same amount of sample i.e. a sensed volume. It is thus possible to calibrate the measurement using one or more standard reference samples containing a known quantity of iron powder, e.g. embedded within epoxy resin. A calibration curve can thus be determined. The calibration data may be stored by the sensor apparatus or where the obtained information is transmitted to an external processor may be stored at the external processor. It has been found that for typical ferrous particles the change in inductance essentially scales linearly with the content of ferrous particles within the sensing region. To achieve best accuracy it is desirable for the sample to be uniformly distributed within the sensing region. For a relatively short sensing coil 21 it is relatively easy to provide a longer sample such that the ends of the sample contribute very little to the measurement. Shaking the sample prior to the measurement may also help achieve a more uniform distribution.

A long solenoidal coil has an apparent advantage over a short coil for this first application, where a sample containing many distributed particles is to be measured, because the fractional change in the signal can be larger, i.e. the sample can be made to extend the length of the coil and thus more coil turns can be provided adjacent to the sample. However, it has been found that in reality the major factor that limits sensitivity is the mechanical stability of the coil which depends on the materials and construction. As discussed above, a planar printed coil is better from a constructional viewpoint than a wound solenoid. The materials used to form the substrate may be chosen to give a fairly similar performance to typical materials used to form the bobbin. The sensitivity of the planar coils described herein may therefore be at least comparable to that of a conventional solenoidal coil.

While this example has been given in relation to determining a total content of ferrous particles per unit volume, as these are most commonly found in samples, the system will determine a total content of any ferromagnetic particles sensed. Thus a total content of ferromagnetic particles, including or consisting of ferrous particles per unit volume will be determined. Suitable calibration should be used based upon the metal material(s) expected to be present in the sample.

A second application is the counting and optionally categorisation of individual particles passing through the aperture 23, e.g. in a continuous fluid flow. Further details of this application are given below

The frequency of the alternating current supplied by the power supply 41 to drive the sensing coil 21, and thus to generate the magnetic field may affect the sensitivity of the measurement. A suitable frequency may be chosen based on the type or size of particles to be detected and/or the type of measurement to be performed. For instance, where the sensor apparatus is used to measure the total content, or to quantify the size, of ferrous or ferromagnetic metal wear particles in a sample, a typical driving frequency might be less than 1 MHz, for example about 100 kHz. This is because the linear relationship between the change in inductance of the sensing coil 21 and the amount of ferromagnetic e.g. ferrous material in the sample being measured may start to break down at high frequencies, e.g. above 5 MHz. On the other hand, it may be preferable to use higher frequencies, e.g. in the range 1 MHz to 100 MHz, where the sensor apparatus is used to simply count individual metal particles in a flow of sample fluid, as the sensitivity (i.e. ability to detect smaller particles) generally improves at higher frequencies. Furthermore, because the coil impedance depends on the product of frequency and inductance, and so at higher frequencies fewer coil turns are needed to achieve a convenient value of impedance for measuring. Of course, the present invention is not limited to these frequencies, and other suitable frequencies may be used.

It will be understood that other techniques may be used to detect metal particles not employing a bridge circuit. Although most of the discussion herein is in the context of sensing a change in impedance of the coil, other suitable measuring techniques will also be apparent to the skilled person that may also be used. For instance, it is also contemplated that the sensing means may measure a change in coil inductance more directly, for instance, by monitoring the resonant frequency of an LC circuit comprising the conductive coil and a known capacitor. Other ways of measuring the interactions between metal particles and an applied magnetic field will also be apparent to the skilled person.

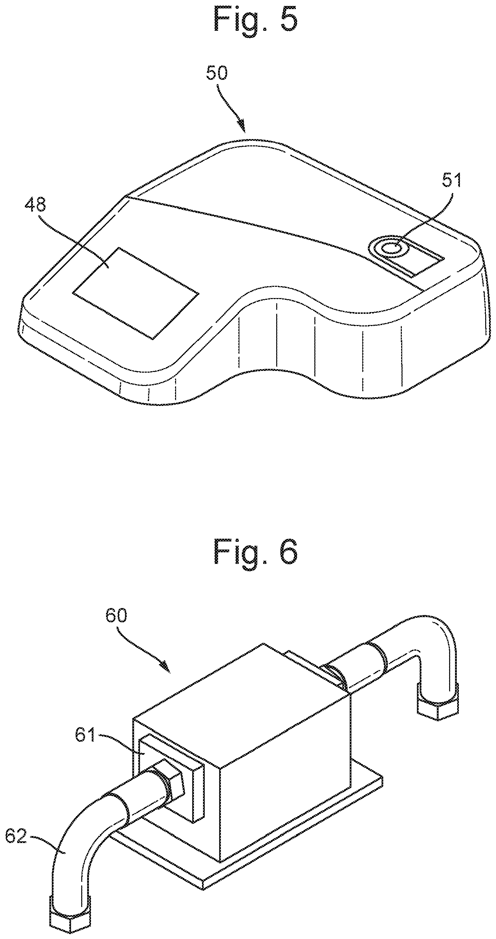

Fig, 5 shows an example of a sensor unit incorporating a sensor apparatus of the type described herein. A housing 50 is provided with a display 48 and an orifice 51 for receiving a sample tube. The orifice 51 is coaxial with the aperture in the substrate and hence the sensing coil. The Fig. 5 product is particularly suited to the first application described above where the aim is to determine the total content of ferrous material per unit volume in a sample. The unit shown in Fig. 5 is portable and self-contained, so that all of the components, including the processor, are provided within the housing 50 alongside the sensor apparatus.

A second application of the type of sensor apparatus described above is the counting and categorisation of individual particles passing through the aperture 23, e.g. in a continuous fluid flow. For instance, the sensor apparatus may be mounted onto a flow pipe to monitor machine wear debris in situ without having to extract any fluid. As above, whenever metal particles pass into the sensing region of the coil, their presence will result in a change in impedance of the sensing coil 21 and the coil impedance can thus be monitored to assess the rate of metal wear debris generation. Furthermore, by making certain assumptions it is possible to correlate a change in impedance with size and/or type of metal particle. For instance, the same principles used in the first application can be employed to determine the size of ferrous particles passing through the aperture (assuming that the measured signal corresponds to a single metal particle). Additionally, by analysing the shape of the measured impedance change, e.g. by analysing the in-phase and out-of-phase contributions to the measured signal, it is possible to determine information about the magnetic properties of the particles. Particularly, it is possible to determine whether the particle is ferromagnetic or non-ferromagnetic. In order to accurately correlate the change in impedance with the size and/or type of metal particle flowing through the aperture 23 it may be necessary to assume that the measured signal is associated with a single particle. The validity of this assumption may be improved by controlling the flow rate. In the present invention, because the coil 21 is essentially planar and the sampling volume is relatively short, this approximation is more likely to be valid. With conventional coil arrangements like that shown in Fig. 1 the signal may become blurred by the presence of other metal particles in the sample fluid. For example, engine fluids will typically contain tens of thousands of relatively fine particles resulting from normal wear. These are typically too small to be detected individually, but together constitute background noise. These particles are generally distributed fairly uniformly within the fluid and so the size of the background noise increases with the sampling volume. The Fig. 2 coil arrangement may therefore provide a lower signal to noise ratio than an extended solenoidal coil like that shown in Fig. 1. Additionally, because the sampling volume is shorter than would be the case for a solenoidal coil, the signal will be sharper and the sensor may provide a relatively high resolution and/or may be compatible with higher flow rates. In one exemplary embodiment the background particles may have a typical size of less than about 10 microns, and the sensitivity of the sensor apparatus may be such that particles having a typical size of greater than around 40 or 50 microns are detected. However, the sensor apparatus may have a sensitivity for detecting particles of any suitable size for a particular application.

It will be appreciated that the larger the sampling volume, the greater the probability that two or more particles will be sensed at the same time and incorrectly classified as a single larger particle. The ability to provide a smaller sampling volume that is associated with the use of a printed coil may help to improve the accuracy with which individual particles may be counted, and, where appropriate, sized. Improved reliability in counting may be obtained even at higher particle concentrations. This makes the apparatus of the present invention particularly useful for detecting smaller particles as they are typically present at relatively high concentrations.

To give an indication of the distribution of particle sizes within a typical flow of lubricating fluid or engine oil, then it is noted that even new oil may contain over a hundred particles per millilitre greater than about 14 microns in size, and more than a thousand particles per millilitre greater than about 4 microns in size. Used oil may contain an order of magnitude greater number of particles per millilitre. During normal engine wear, metal particles in around the 1-15 microns range are typically generated. The onset of abnormal wear will tend to produce larger particles, e.g. in the 25 - 100 microns range.

It will be appreciated that the distribution will be skewed towards small particles, i.e. that smaller particles will be vastly more common than larger ones. This is partly because smaller particles are generated constantly by normal wear, partly because any larger particles will eventually break up into smaller particles and partly because larger particles (> about 10 microns) may be filtered out by the oil filtration system, which may be insensitive to smaller particles. Furthermore, larger, heavier particles tend to settle out of the oil very quickly e.g. a 100 micron steel particle may settle in a matter of seconds. Whilst large particles tend to settle, break up or be filtered out, smaller particles will continue to circulate in suspension within the fluid. This removal or larger particles from the flow is problematic for monitoring machine wear debris by analysing the metal particle content of the lubricating oil or grease. For instance, for offline (lab) tests it is difficult to know whether the extracted sample is really indicative of the distribution of particles being generated at the moving parts in the engine. Naturally, this is also problematic for online sensors that are only capable of detecting relatively large particles, as again, unless the sensor is located in close proximity to the source of the fault, any particles that are large enough to be detected, and would indicate abnormal wear, may not reach the sensor.

As described above, the presence of many thousands of small particles within the fluid, which may be too small to be individually detected but whose aggregate signal and fluctuations thereof is a source of noise that limits the ability of the sensor to count large particles, limits the size of particles that can be detected by a sensor apparatus. Naturally, the size of this noise increases with the size of the sensing region of the sensor apparatus. Thus, it will be appreciated that the use of a relatively small or planar sensor apparatus, substantially of the type described above, is particularly advantageous for this second application, because the relatively small sensing region which may be provided (e.g. compared to a conventional wound coil arrangement) allows the detection of much smaller particles with a higher signal to noise ratio. Thus, a relatively small sensor apparatus with a relatively small sensing volume is capable of detecting particles that remain in suspension and give early indications of developing problems.

An example of a sensor unit that may incorporate a sensor apparatus as described herein for use in the second application is shown in Fig. 6. Here, the sensor unit is arranged to be mounted between sections of or around a flow pipe so that sample fluid may continuously flow through the aperture of the sensor apparatus. The housing 60 may contain a flow conduit disposed coaxially within the aperture and extending between opposite sides of the housing parallel to the substrate. Connectors 61 may be provided at the ends of the flow conduit and/or on the sides of the housing to enable the housing 60 to be connected between sections of an existing, external flow pipe 62. The sensor apparatus shown in Fig. 6 connects or bridges between a first upstream and second downstream portion of a pipe, and thus forms part of a continuous flow path. The sensor unit being mounted onto a flow pipe is particularly suited to the second application described above where the aim is to count and/or categorise individual metal particles flowing through the sensor. The unit shown in Fig. 6 does not provide a display to the user, but instead provides an output to a remote display. Preferably the output is provided wirelessly to the remote display. The unit can thus be left in situ and continuously monitored by a remote user. The output may comprise a count of particles, and may further comprise indicating a change in count e.g. over a period of time.

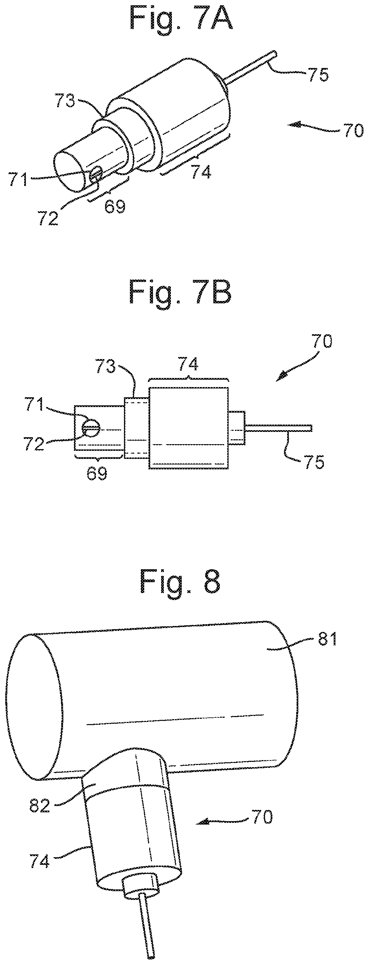

Figures 7A and B show a perspective and cross sectional view respectively of another example of a sensor unit intended for use in the second application described above. A portion of the sensor unit shown in Figures7A and B is arranged to be immersed within a dynamic flow of fluid for counting individual metal particles dispersed within the fluid and passing through the sensor. The sensor apparatus of the sensor unit of Figures 7A and B may thus be mounted within a pipe for in situ i.e. online measurements.

The sensor unit 70 comprises a first portion 69 arranged to be immersed within a flow of fluid and including a passage 71 through which a portion of the total sample fluid flow within a pipe passes. At least a portion of a sensor apparatus having a coil arrangement of the type described above e.g. in Figures 2 or 3, having one or more coil units printed onto a flexible substrate around an aperture, is located within the first portion so that fluid passing through the passage 71 then passes through the aperture (i.e. into the sensing region) of the sensor apparatus so that a determination of the presence of individual metal particles within the fluid can be made. This may be achieved in various manners. For instance, the passage may be defined by a tube, with the aperture surrounding the tube. In this way, the tube, and fluid passing therealong, will flow through sensing region of the sensor apparatus. Alternatively, the portion of the substrate defining the walls of the aperture of the sensor apparatus may at least in part define the flow passage 71.

A reference coil may also be provided and positioned within the sensor apparatus 70 such that it is exposed to substantially the same temperature and pressure conditions within the pipe but arranged so that metal particles within the fluid flow do not pass through the reference coil. For instance, a baffle may be provided as part of the sensor body to prevent the metal particles passing through the reference coil. Alternatively, the reference coil may be arranged so that it does not encounter i.e. is sealed off from or not immersed within the fluid flow. The reference coil may generally be provided on the same substrate as the sensing coil, and spaced apart from the sensing coil by approximately a coil diameter, substantially as shown in Fig. 3.

The sensor unit 70 also comprises a second portion 74 that is arranged to be outside of the fluid flow i.e. outside of the pipe, and to house the electronics or processing means of the sensor unit 70. The second portion 74 further includes any connections necessary to transmit the sensed information to a user or to an external processing unit. For instance, in the embodiment illustrated in Figures 7A and B the second portion 74 includes a wired external connection.

In order to facilitate its mounting within a pipe, the sensor unit shown in Figures 7A and B also contains a collar or mounting ring 73 that may, for example, be threaded to allow the sensor 70 to be screwed into a suitable opening in the pipe. The mounting ring 73 and/or the shoulder between the second portion 74 and the mounting ring 73 provide a fluid-tight seal around the mounting.

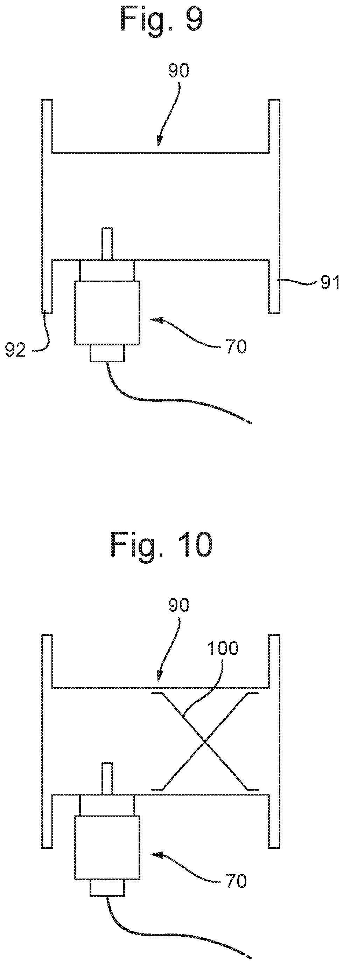

Fig. 8 illustrates the sensor unit 70 mounted in situ within a pipe 81. In the arrangement illustrated in Fig. 8 a collar 82 for receiving the sensor unit has been welded onto the pipe 81. The collar 82 on the pipe 81 engages the mounting ring 73 of the sensor unit. For instance, the collar 82 and the mounting ring 73 may be correspondingly threaded, or otherwise connected. The shoulder between the second portion 74 and the mounting ring 73 abuts the end of the collar 82. However, it will be appreciated that any other suitable arrangements for mounting the sensor unit 70 onto a pipe may be used. For example, Fig. 9 shows an arrangement where the sensor unit 70 is provided integrally with a section of pipe 90. The pipe section 90 terminates in mounting flanges 91, 92 to enable it to be mounted to adjacent pipe sections. The pipe section 90 can thus be mounted as a unit within an existing flow pipe. Providing the sensor unit 70 as part of a pipe section 90 may advantageously allow further components to be positioned adjacent to the sensor apparatus. For example, in Fig. 10, which illustrates an alternative embodiment in which the sensor unit 70 is provided mounted to a section of pipe, a stator mixer 100 is provided upstream of the sensor apparatus. The use of an upstream mixer 100 may help to ensure that the fraction of the fluid passing into and sensed by the sensor unit 70 more accurately reflects the total composition of the fluid, i.e. the dispersion of metal particles within the fluid and that the measurement is not skewed by the (radial) position of the sensor apparatus in the pipe due to e.g. variations in flow profile across the cross section of the pipe.

It will be appreciated that the sensor unit 70 shown in Figs. 7-10 is arranged to sample only a portion or fraction of the fluid flow through the pipe or pipe section, the sampled portion being defined by the diameter of the passage 71. This is by contrast to arrangements where a larger diameter sensor is mounted around the circumference of the pipe and used to monitor the entire flow. It will also be appreciated that the sensor unit shown in Fig. 6 may also be arranged to sample only a fraction of a larger flow, e.g. where the flow pipe 62 is part of bypass arrangement. As explained above, a flow of lubricating oil or engine grease will generally contain a distribution of particle sizes including a vast number of relatively small particles. By arranging for the sensor apparatus to see only a fraction of the fluid flow, the size i.e. diameter of the sensor apparatus can be reduced (at least relative to the pipe diameter), thus reducing the sensing volume and background noise further, again allowing the detection of much smaller particles. It has been found that the small bore sensors which may be provided in accordance with the invention may be sensitive enough to provide a count that is indicative of wear rate. In contrast, larger bore sensors that are mounted around the flow pipe and thus sample the whole cross-sectional flow may only be capable of detecting particularly large particles that may only be present in the case of imminent catastrophic failure. Furthermore, such large particles may drop out of suspension relatively quickly or be filtered out, before reaching the sensor. Thus, it will be appreciated that sampling only a fraction of the total flow may advantageously allow better detection of developing faults, i.e. particle sizes indicative of the onset of abnormal wear.

By way of example only, relating to a sensor for use in the context of monitoring metal particles within a marine engine, a flow passage through the sensor unit, which may correspond to the diameter of the aperture in the substrate in accordance with the invention, may be within the range about 3-5 mm, and ideally towards the lower end of this range to provide a relatively high sensitivity. However, in some circumstances it may be advantageous to use a larger diameter, e.g. towards the upper end of this range, or higher, so that a larger proportion of the flow can be sampled. This is by contrast to larger bore sensors in this context, which may have a diameter of e.g. 25 or 38 mm, that are mounted around the flow pipe and thus sample the whole cross-sectional flow. The typical detection limit for a large bore (i.e. > 25 mm) sensor may be around 250 microns for ferrous particles and between about 500-750 microns for non-ferrous particles. These dimensions are merely exemplary, and the sensor of the present invention may be arranged to have a flow passage/aperture with diameter selected over a wide range of values, depending upon the particular application in which it is to be used. Generally, there is a balance between the size of the passage (or aperture), the flow speed and the measurement interval to ensure that a count of particles per volume can be obtained with a sufficiently high confidence level. The uncertainty in the count will generally vary as the square root of the total number of particles counted in a given period.

In this context, where the particles being detected are entrained within a continuous fluid flow, and may remain in suspension for multiple passes through the sensor apparatus, it is desirable to be able to count not just the absolute number of particles flowing through the sensor over time (e.g. the counts per minute or hour), but also to determine the count as a function of volume. For instance, outputting the count as a function of volume allows more accurate comparisons to be made between measurements obtained using an online sensor unit of the type described above with offline lab results, or with other particle counting methods. Particularly, obtaining the count as a function of volume allows for consistent measurements to be obtained under different operating conditions e.g. different pumping speeds. For example, if the output was provided only as a count as a function of time, a sharp increase in the number of metal particles being counted could be caused by an increase in flow rate, rather than an increase in wear.

In order to achieve this, a flow sensor 72 is provided integrated within the sensor unit 70 (e.g. in accordance with the embodiments of any one of Figures 7 to 10) for determining the volume of fluid that is measured by the sensing coil within a test period. The volume may for instance be determined using the cross-sectional area of the aperture of the sensor apparatus and a measured flow velocity through this aperture. In the Fig. 7A and B embodiment the flow sensor 72 is located within the passage 71, i.e. within the same flow passage as the sensing region of the coil, such that the flow sensor 72 measures the flow velocity of the same fraction of fluid that is to be measured by the sensing coil. Typically, the flow sensor 72 may be mounted just upstream or downstream of the coil sensor. It will be appreciated that the flow sensor 72 may be secured via a suitable adaptor to the substrate upon which the coil sensor is printed, or could be provided as part of the substrate. Alternatively, the flow sensor 72 may be secured within the flow passage, but not directly to the substrate. Other suitable ways of mounting the flow sensor 72 will be apparent to the skilled person. The flow sensor 72 should be located in close proximity to the sensing coil to ensure that the measured flow velocity can be taken to be indicative of the fraction of the flow that is sensed by the coil sensor. This is important because the sampling fraction may be affected by variations in the flow profile across the width of the pipe. It will be appreciated however that the flow sensor 72 may be positioned sufficiently far from the sensing coil to minimise any interactions between any metallic components of the flow sensor and the magnetic field of the coil sensor. That is, the flow sensor 72 may be substantially outside of the sensing region of the coil sensor. By way of example only, for a printed coil sensor of the type described above having an aperture of around 3-5 mm diameter, the flow sensor may be a few millimetres away from the coil sensor; however, the most appropriate distance will of course depend on the size of the sensor apparatus. Generally, there may be some compromise between keeping the coil sensors small, and hence sensitive to smaller particles, and the risk of there being significant interactions with the flow sensor.

There are various known ways of measuring flow that are suitable for use with these embodiments. For example, a preferred embodiment would be the use of planar thermal mass flow anemometer-type sensor that senses the cooling effect of the flow. A suitable planar thermal mass flow sensor may be that available from Innovative Sensor Technology designated as the FS5 Thermal Mass Flow Sensor. This type of planar sensor is shown in Fig. 7A and B, with the plane of the flow sensor 72 being oriented in the radial direction when mounted in the pipe. However, the orientation of the flow sensor 72 is not particularly important. It will also be appreciated that any other suitable flow sensors may also be used. For instance, a linear or hotwire type anemometer sensor may also be used. As another example, a capillary thermal mass flow sensor including a central heater with upstream and downstream temperature sensors for comparing the heat transfer with and against the direction of flow may be used. Other (non-thermal) sensors may also be used to measure the flow. For instance, a pressure sensor may be used to measure a pressure drop between two points. For example, an orifice type sensor measuring the pressure drop across the coil may be used. As another example, a venturi-type may be used to measure the pressure difference between the coil, which provides the throat of the venturi sensor, and the main flow. Other types of sensors, including simple rotating impeller type sensors may also suitably be used.

Whilst the embodiment described above in relation to Figs. 7-10 is advantageously implemented using a sensor apparatus of the type described above in relation to and shown in Figs. 2 and 3 especially, it will be understood that the technique of counting particles within a dynamic flow of fluid and outputting the count as a function of volume is not limited to this type of sensor. For instance, a conventional wound bobbin-type coil sensor like that shown in Fig. 1 could also be used in conjunction with a flow sensor in a similar manner. However, it will be appreciated that the advantages at least in sensitivity obtainable using a sensor including one or more coil portions printed around an aperture are particularly useful in the context of the second main application described herein.

It will be understood that any of the sensor units described herein may internally process the output signal generated by the sensing means, or an output signal indicative of the output of the sensing means, or may transmit such signals to a remote processor. It will also be understood that any of the sensor units described herein may comprise a display, or may provide data to a remote system for display. For any of the sensor units described herein data may be transmitted to a remote system wirelessly, via a cable or via a removable storage means. The sensor units Figs. 5-10 are not intended to be limiting in these respects and merely illustrate a number of possible arrangements. It is also noted that the features described above in relation to Fig. 4, including any modifications to what is illustrated in Fig. 4, are compatible with both the sensor unit of Fig. 5 and the sensor units of Fig. 6-10.

Embodiments of the present invention have been described with respect to determining a total quantity (mass) of ferromagnetic e.g. ferrous particles, e.g. per given/unit volume of an extracted sample. Typically the ferromagnetic particles will be ferrous. The total quantity may then be one of ferrous particles. However, where non ferrous ferromagnetic particles are present they may contribute to the sensed interaction, and hence to the determined total quantity of ferromagnetic particles. Although the present invention has been described with reference to preferred embodiments, it will be understood by those skilled in the art that various changes in form and detail may be made without departing from the scope of the invention as set forth in the accompanying claims.

## Claims

1. A sensor apparatus for use in obtaining information relating to the presence of metal particles within a sample of a substance to be tested, the apparatus comprising:
a printed circuit board (PCB) defining a substrate (22) comprising an aperture (23) for receiving a sample of a substance to be tested;
magnetic field generating means for generating a magnetic field for application to a sample received in the aperture (23) in use; and
sensing means for sensing the result of interaction between the generated magnetic field and a sample received in the aperture (23) in use, the sensed result of the interaction being usable to determine a quantity of ferromagnetic particles (25) in the sample;
wherein the substrate (22) includes an electrically conductive coil (21) printed thereon that circumferentially surrounds said aperture (23) for receiving the sample, the electrically conductive coil (21) forming part of said sensing means.

2. The sensor apparatus of claim 1, wherein the sample is an extracted sample contained within a sample container, such as a test tube.

3. The sensor apparatus of claim 1, wherein the sample is a dynamic sample defined by a volume of fluid which flows through the aperture between first and second times.

4. The sensor apparatus of any of claims 1, 2 or 3, further comprising a reference component for calibrating or balancing said sensing means, wherein said reference component comprises a reference coil printed on the substrate and/or further comprising electrostatic shielding disposed between the conductive coil of said sensing means and the aperture wherein said electrostatic shielding comprises an incomplete ring printed on said substrate.

5. The sensor apparatus of any preceding claim wherein at least the coil surrounding the aperture and forming part of the sensing means comprises multiple coil portions printed at different respective levels of the substrate; or wherein the substrate comprises multiple substrate layers, and wherein at least the coil surrounding the aperture and forming part of the sensing means comprises coil portions printed on more than one layer of the substrate, each coil portion being printed at a different respective level of the substrate.

6. The sensor apparatus as claimed in any preceding claim wherein the coil surrounding the aperture is a spiral coil.

7. The sensor apparatus of any preceding claim wherein the PCB substrate further comprises the magnetic field generating means.

8. The sensor apparatus as claimed in any preceding claim, wherein the printed conductive coil circumferentially surrounding said aperture that forms part of the sensing means also forms part of said magnetic field generating means.

9. The sensor apparatus as claimed in any preceding claim, wherein said sensing means is arranged to sense a change in impedance of the conductive coil resulting from said interaction between the sample fluid and the generated magnetic field.

10. The sensor apparatus of any preceding claim wherein the sensor apparatus comprises means for generating data indicative of the sensed result of the interaction between the sample and the generated magnetic field and for providing the generated data indicative of the sensed result to one or more of output means for outputting information to a user based on the data indicative of the sensed result, storage means for storing data based on the data indicative of the sensed result, and processing means for using the data indicative of the sensed result in obtaining information relating to the presence of metal particles within the sample.

11. The sensor apparatus of any preceding claim further comprising processing means for using the sensed result of said interaction to determine a quantity of ferromagnetic particles within the sample.

12. A system comprising the sensor apparatus of any one of claims 1 to 10 and processing means remote from the sensor apparatus for using the sensed result of said interaction to determine a quantity of ferromagnetic particles within the sample.

13. The sensor apparatus of claim 11 or the system of claim 12, wherein the quantity is a total content of the particles per unit volume.

14. A sensor unit comprising a sensor apparatus as claimed in any of claims 1 to 11 or 13, wherein the sensor unit comprises a housing, wherein said housing comprises an opening coaxial with said aperture of the sensor apparatus to enable a sample to be tested to be received within the aperture

15. A method of obtaining information relating to the presence of metal particles within a sample using the sensor apparatus or system of any preceding claim, wherein the method comprises:
locating a sample of a substance to be tested such that it is received in said aperture (23);
using the magnetic field generating means to generate a magnetic field;
using the sensing means to sense the result of interaction between the sample and the applied magnetic field; and
using the sensed result of interaction between the sample and the generated magnetic field to determine a quantity of ferromagnetic particles (25) in the sample.

## Patentansprüche

1. Sensoreinrichtung zur Verwendung bei der Erlangung von Informationen in Bezug auf das Vorhandensein von Metallteilchen innerhalb einer Probe einer zu prüfenden Substanz, wobei die Einrichtung Folgendes umfasst:
eine Leiterplatte (PCB), die ein Substrat (22) definiert, das eine Öffnung (23) zur Aufnahme einer Probe einer zu prüfenden Substanz umfasst;
Magnetfelderzeugungsmittel zum Erzeugen eines Magnetfeldes zum Anlegen an eine in der verwendeten Öffnung (23) aufgenommene Probe; und
Erfassungsmittel zum Erfassen des Ergebnisses der Wechselwirkung zwischen dem erzeugten Magnetfeld und einer in der verwendeten Öffnung (23) aufgenommenen Probe, wobei das erfasste Ergebnis der Wechselwirkung verwendbar ist, um eine Menge an ferromagnetischen Teilchen (25) in der Probe zu bestimmen;
wobei das Substrat (22) eine darauf gedruckte elektrisch leitfähige Spule (21) einschließt, welche die Öffnung (23) zur Aufnahme der Probe in Umfangsrichtung umgibt, wobei die elektrisch leitfähige Spule (21) einen Teil der Erfassungsmittel bildet.

2. Sensoreinrichtung nach Anspruch 1, wobei die Probe eine extrahierte Probe ist, die innerhalb eines Probenbehälters, wie beispielsweise einem Reagenzglas, enthalten ist.

3. Sensoreinrichtung nach Anspruch 1, wobei die Probe eine dynamische Probe ist, die durch ein Fluidvolumen definiert ist, das zwischen einem ersten und zweiten Mal durch die Öffnung fließt.

4. Sensoreinrichtung nach einem der Ansprüche 1, 2 oder 3, die weiter eine Referenzkomponente zum Kalibrieren oder Ausbalancieren der Erfassungsmittel umfasst, wobei die Referenzkomponente eine auf das Substrat gedruckte Referenzspule umfasst und/oder weiter eine zwischen der leitfähigen Spule der Erfassungsmittel und der Öffnung angeordnete elektrostatische Abschirmung umfasst, wobei die elektrostatische Abschirmung einen unvollständigen Ring umfasst, der auf das Substrat gedruckt ist.

5. Sensoreinrichtung nach einem der vorstehenden Ansprüche, wobei mindestens die Spule, welche die Öffnung umgibt und einen Teil der Erfassungsmittel bildet, mehrere Spulenabschnitte umfasst, die auf unterschiedlichen jeweiligen Ebenen des Substrats gedruckt sind; oder wobei das Substrat mehrere Substratschichten umfasst, und wobei mindestens die Spule, welche die Öffnung umgibt und einen Teil der Erfassungsmittel bildet, Spulenabschnitte umfasst, die auf mehr als eine Schicht des Substrats gedruckt sind, wobei jeder Spulenabschnitt auf einer unterschiedlichen jeweiligen Ebene des Substrats gedruckt ist.

6. Sensoreinrichtung nach einem der vorstehenden Ansprüche, wobei die Spule, welche die Öffnung umgibt, eine Spiralspule ist.

7. Sensoreinrichtung nach einem der vorstehenden Ansprüche, wobei das PCB-Substrat weiter die Magnetfelderzeugungsmittel umfasst.

8. Sensoreinrichtung nach einem der vorstehenden Ansprüche, wobei die gedruckte leitfähige Spule, welche die Öffnung in Umfangsrichtung umgibt und einen Teil der Erfassungsmittel bildet, auch einen Teil der Magnetfelderzeugungsmittel bildet.

9. Sensoreinrichtung nach einem der vorstehenden Ansprüche, wobei die Erfassungsmittel angeordnet sind, um eine Änderung der Impedanz der leitfähigen Spule zu erfassen, die sich aus der Wechselwirkung zwischen dem Probenfluid und dem erzeugten Magnetfeld ergibt.

10. Sensoreinrichtung nach einem der vorstehenden Ansprüche, wobei die Sensoreinrichtung Mittel zum Erzeugen von Daten, die das erfasste Ergebnis der Wechselwirkung zwischen der Probe und dem erzeugten Magnetfeld anzeigen, und zum Bereitstellen der erzeugten Daten, die das erfasste Ergebnis anzeigen, an eine oder mehrere Ausgabemittel zum Ausgeben von Informationen an einen Benutzer, basierend auf den das erfasste Ergebnis anzeigenden Daten, Speichermittel zum Speichern von Daten, basierend auf den das erfasste Ergebnis anzeigenden Daten, und Verarbeitungsmittel zum Verwenden der Daten, die das erfasste Ergebnis anzeigen, bei der Erlangung von Informationen in Bezug auf das Vorhandensein von Metallteilchen innerhalb der Probe, umfasst.

11. Sensoreinrichtung nach einem der vorstehenden Ansprüche, die weiter Verarbeitungsmittel zur Verwendung des erfassten Ergebnisses der Wechselwirkung umfasst, um eine Menge an ferromagnetischen Teilchen innerhalb der Probe zu bestimmen.

12. System, umfassend die Sensoreinrichtung nach einem der Ansprüche 1 bis 10 und Verarbeitungsmittel, die von der Sensoreinrichtung entfernt sind, zur Verwendung des erfassten Ergebnisses der Wechselwirkung, um eine Menge von ferromagnetischen Teilchen innerhalb der Probe zu bestimmen.

13. Sensoreinrichtung nach Anspruch 11 oder das System nach Anspruch 12, wobei die Menge ein Gesamtgehalt der Teilchen pro Volumeneinheit ist.

14. Sensoreinheit, umfassend eine Sensoreinrichtung nach einem der Ansprüche 1 bis 11 oder 13, wobei die Sensoreinheit ein Gehäuse umfasst, wobei das Gehäuse eine Öffnung koaxial zur Öffnung der Sensoreinrichtung umfasst, so dass eine zu prüfende Probe innerhalb der Öffnung aufgenommen werden kann.

15. Verfahren zur Erlangung von Informationen in Bezug auf das Vorhandensein von Metallteilchen innerhalb einer Probe unter Verwendung der Sensoreinrichtung oder des Systems nach einem der vorstehenden Ansprüche, wobei das Verfahren Folgendes umfasst:
Lokalisieren einer Probe einer zu prüfenden Substanz, so dass sie in der Öffnung (23) aufgenommen wird;
Verwenden der Magnetfelderzeugungsmittel, um ein Magnetfeld zu erzeugen;
Verwenden der Erfassungsmittel, um das Ergebnis der Wechselwirkung zwischen der Probe und dem angelegten Magnetfeld zu erfassen; und
Verwenden des erfassten Ergebnisses der Wechselwirkung zwischen der Probe und dem erzeugten Magnetfeld, um eine Menge an ferromagnetischen Teilchen (25) in der Probe zu bestimmen.

## Revendications

1. Appareil détecteur pour une utilisation dans l'obtention d'informations se rapportant à la présence de particules de métal à l'intérieur d'un échantillon d'une substance à évaluer, l'appareil comprenant :
une carte à circuit imprimé (PCB) définissant un substrat (22) comprenant une ouverture (23) pour recevoir un échantillon d'une substance à évaluer ;
un moyen de production de champ magnétique pour produire un champ magnétique pour application à un échantillon reçu dans l'ouverture (23) lors de l'utilisation ; et
un moyen de détection pour détecter le résultat d'interaction entre le champ magnétique produit et un échantillon reçu dans l'ouverture (23) lors de l'utilisation, le résultat détecté de l'interaction étant utilisable pour déterminer une quantité de particules ferromagnétiques (25) dans l'échantillon ;
dans lequel le substrat (22) inclut une bobine électriquement conductrice (21) imprimé sur ce dernier qui entoure de manière circonférentielle ladite ouverture (23) pour recevoir l'échantillon, la bobine électriquement conductrice (21) formant une partie dudit moyen de détection.

2. Appareil détecteur selon la revendication 1, dans lequel l'échantillon est un échantillon extrait contenu à l'intérieur d'un conteneur d'échantillon, comme un tube à essais.

3. Appareil détecteur selon la revendication 1, dans lequel l'échantillon est un échantillon dynamique défini par un volume de fluide qui s'écoule à travers l'ouverture entre des premier et second instants.

4. Appareil détecteur selon l'une quelconque des revendications 1, 2 ou 3, comprenant en outre un composant de référence pour étalonner ou équilibrer ledit moyen de détection, dans lequel ledit composant de référence comprend une bobine de référence imprimée sur le substrat et/ou comprenant en outre un blindage électrostatique disposé entre la bobine conductrice dudit moyen de détection et l'ouverture dans laquelle ledit blindage électrostatique comprend un anneau incomplet imprimé sur ledit substrat.

5. Appareil détecteur selon l'une quelconque des revendications précédentes dans lequel au moins la bobine entourant l'ouverture et formant une partie du moyen de détection comprend des portions de bobine multiples imprimées à différents niveaux respectifs du substrat; ou dans lequel le substrat comprend des couches de substrat multiples, et dans lequel au moins la bobine entourant l'ouverture et formant une partie du moyen de détection comprend des portions de bobine imprimées sur plus d'une couche du substrat, chaque portion de bobine étant imprimée à un niveau respectif différent du substrat.

6. Appareil détecteur selon l'une quelconque des revendications précédentes dans lequel la bobine entourant l'ouverture est une bobine en spirale.

7. Appareil détecteur selon l'une quelconque des revendications précédentes dans lequel le substrat PCB comprend en outre le moyen de production de champ magnétique.

8. Appareil détecteur selon l'une quelconque des revendications précédentes, dans lequel la bobine conductrice imprimée entourant de manière circonférentielle ladite ouverture qui forme une partie du moyen de détection forme également une partie dudit moyen de production de champ magnétique.

9. Appareil détecteur selon l'une quelconque des revendications précédentes, dans lequel ledit moyen de détection est agencé pour détecter un changement d'impédance de la bobine conductrice résultant de ladite interaction entre le fluide échantillon et le champ magnétique produit.

10. Appareil détecteur selon l'une quelconque des revendications précédentes, dans lequel l'appareil détecteur comprend un moyen pour produire des données indicatives du résultat détecté de l'interaction entre l'échantillon et le champ magnétique produit et pour fournir les données produites indicatives du résultat détecté à un ou plus de moyens de sortie pour sortir des informations à un utilisateur sur la base des données indicatives du résultat détecté, un moyen de stockage pour stocker des données basées sur les données indicatives du résultat détecté, et un moyen de traitement pour utiliser les données indicatives du résultat détecté en obtenant des informations se rapportant à la présence de particules de métal à l'intérieur de l'échantillon.

11. Appareil détecteur selon l'une quelconque des revendications précédentes, comprenant en outre un moyen de traitement pour utiliser le résultat détecter de ladite interaction pour déterminer une quantité de particules ferromagnétiques à l'intérieur de l'échantillon.

12. Système comprenant l'appareil détecteur selon l'une quelconque des revendications 1 à 10 et un moyen de traitement distant de l'appareil détecteur pour utiliser le résultat détecté de ladite interaction pour déterminer une quantité de particules ferromagnétiques à l'intérieur de l'échantillon.

13. Appareil détecteur selon la revendication 11 ou système selon la revendication 12, dans lequel la quantité est une teneur totale en particules par volume unitaire.

14. Unité détectrice comprenant un appareil détecteur selon l'une quelconque des revendications 1 à 11 ou 13, dans laquelle l'unité détectrice comprend un logement, dans lequel ledit logement comprend une ouverture coaxiale avec ladite ouverture de l'appareil détecteur pour permettre à un échantillon à évaluer d'être reçu à l'intérieur de l'ouverture.

15. Procédé d'obtention d'informations se rapportant à la présence de particules de métal à l'intérieur d'un échantillon en utilisant l'appareil détecteur ou le système selon l'une quelconque des revendications précédentes, dans lequel le procédé comprend :
le placement d'un échantillon d'une substance à évaluer de sorte qu'il est reçu dans ladite ouverture (23) ;
l'utilisation du moyen de production de champ magnétique pour produire un champ magnétique ;
l'utilisation du moyen de détection pour détecter le résultat d'interaction entre l'échantillon et le champ magnétique appliqué ; et
l'utilisation du résultat détecté d'interaction entre l'échantillon et le champ magnétique produit pour déterminer une quantité de particules ferromagnétiques (25) dans l'échantillon.
